# EUROPEAN PATENT SPECIFICATION

(11) **EP 2 822 494 B1**
(45) Date of publication and mention of the grant of the patent: **03.08.2022**
(21) Application number: 13712400.4
(22) Date of filing: 04.03.2013
(51) Int. Cl.: A61B 50/13, A61B 18/12, A61B 90/98

(54) **GENERATOR ASSEMBLIES FOR NEUROMODULATION THERAPY**
GENERATORANORDNUNG FÜR EINE NEUROMODULATIONSTHERAPIE
Ensembles de générateurs pour la thérapie par neuromulation

(30) Priority: 04.03.2012 US 201261606456 P; 20.04.2012 US 201261636581 P
(43) Date of publication of application: 14.01.2015
(73) Proprietor: Medtronic Ireland Manufacturing Unlimited Company, Dublin 2, D02 T380 (IE)
(72) Inventor: CADOURI, Hadar, Santa Rosa, CA 95403 (US); FRIEDRICHS, Paul, Santa Rosa, CA 95403 (US); SHAH, JignesH, Santa Rosa, CA 95403 (US); TRUDEL, Julie, Santa Rosa, CA 95403 (US); DOERKSEN, Kyle, Santa Rosa, CA 95403 (US); HARBOUN, Michael, Santa Rosa, CA 95403 (US); KING, Simon, Santa Rosa, CA 95403 (US); VONDLE, Dave, Santa Rosa, CA 95403 (US); BALLAKUR, Sowmya, Santa Rosa, CA 95403 (US); WU, Andrew, Santa Rosa, CA 95403 (US); GEORGE, William R., Santa Rosa, CA 95403 (US); LEWIS, Charles Richard Jr., Santa Rosa, CA 95403 (US); MINAMI, Don S., Santa Rosa, CA 95403 (US)
(74) Representative: Zimmermann & Partner Patentanwälte mbB
(86) International application number: PCT/US2013/028886
(87) International publication number: WO 2013/134133

(56) References cited:
- WO-A2-2008/003058
- US-A1- 2002 082 479
- US-A1- 2003 167 074
- US-A1- 2004 152 982
- US-A1- 2006 024 107
- US-A1- 2008 097 563
- US-A1- 2009 013 731
- US-A1- 2009 270 727
- US-B1- 6 447 451

## Description

### TECHNICAL FIELD

The present technology relates generally to neuromodulation therapies. In particular, several embodiments are directed to generator assemblies for neuromodulation therapy

### BACKGROUND

The sympathetic nervous system (SNS) is a primarily involuntary bodily control system typically associated with stress responses. Fibers of the SNS innervate tissue in almost every organ system of the human body and can affect characteristics such as pupil diameter, gut motility, and urinary output Such regulation can have adaptive utility in maintaining homeostasis or in preparing the body for rapid response to environmental factors. Chronic activation of the SNS, however, is a common maladaptive response that can drive the progression of many disease states. Excessive activation of the renal SNS in particular has been identified experimentally and in humans as a likely contributor to the complex pathophysiology of hypertension, states of volume overload (such as heart failure), and progressive renal disease. For example, radiotracer dilution has demonstrated increased renal norepinephrine (NE) spillover rates in patients with essential hypertension.

Cardio-renal sympathetic nerve hyperactivity can be particularly pronounced in patients with heart failure. For example, an exaggerated NE overflow from the heart and kidneys to plasma is often found in these patients. Heightened SNS activation commonly characterizes both chronic and end stage renal disease. In patients with end stage renal disease, NE plasma levels above the median have been demonstrated to be predictive for cardiovascular diseases and several causes of death. This is also true for patients suffering from diabetic or contrast nephropathy. Evidence suggests that sensory afferent signals originating from diseased kidneys are major contributors to initiating and sustaining elevated central sympathetic outflow.

Sympathetic nerves to the kidneys terminate in the blood vessels, the juxtaglomerular apparatus, and the renal tubules. Stimulation of the renal sympathetic nerves can cause increased renin release, increased sodium (Na⁺) reabsorption, and a reduction of renal blood flow. These neural regulation components of renal function are considerably stimulated in disease states characterized by heightened sympathetic tone and likely contribute to increased blood pressure in hypertensive patients. The reduction of renal blood flow and glomerular filtration rate as a result of renal sympathetic efferent stimulation is likely a cornerstone of the loss of renal function in cardio-renal syndrome (i.e., renal dysfunction as a progressive complication of chronic heart failure). Pharmacologic strategies to thwart the consequences of renal efferent sympathetic stimulation include centrally acting sympatholytic drugs, beta blockers (intended to reduce renin release), angiotensin converting enzyme inhibitors and receptor blockers (intended to block the action of angiotensin II and aldosterone activation consequent to renin release), and diuretics (intended to counter the renal sympathetic mediated sodium and water retention). These pharmacologic strategies, however, have significant limitations including limited efficacy, compliance issues, side effects, and others. Accordingly, there is a strong public-health need for alternative treatment strategies. WO2008003058, US2006024107 and US2002082479 refer to prior art documents relevant for the present invention.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure.
FIG. 1 illustrates a neuromodulation system configured in accordance with an embodiment of the present technology.
FIG. 2 illustrates modulating renal nerves with an intravascular neuromodulation system in accordance with an embodiment of the present technology.
FIGS. 3A and 3B are front and back isometric views, respectively, of a generator system configured in accordance with an embodiment of the present invention.
FIGS. 3C and 3D are back isometric views illustrating a generator console being detached from and reattached to a cart of the generator system of FIGS. 3A and 3B in accordance with an embodiment of the present invention.
FIG. 3E is an isometric view of the generator console of FIGS. 3C and 3D.
FIGS. 3F and 3G are isometric back views of the generator console of FIGS. 3C and 3D.
FIG. 3H is a schematic top view of a catheter coupled to a cable interface of the generator console of FIGS. 3C and 3D in accordance with an embodiment of the present invention.
FIG. 3I is a perspective view of the generator system of FIGS. 3A-3G in a clinical environment in accordance with an embodiment of the present invention.
FIGS. 3J and 3K are front and back isometric views of a generator system configured in accordance with another embodiment of the present invention.
FIGS. 4A-4C are a series of isometric views of a generator system not showing all features of the claimed invention.
FIG. 4D is an enlarged isometric view of a portion of the generator system of FIGS. 4A-4C.
FIG. 4E is an exploded isometric view of a generator assembly of the generator system of FIGS. 4A-4D.
FIG. 4F is an isometric view of the generator assembly of FIG. 4E mounted to a support structure.
FIG. 4G is a perspective view of the generator assembly of FIGS. 4E and 4F in a clinical environment.
FIGS. 5A and 5B are isometric views of a generator system not showing all features of the claimed invention.
FIG. 5C is a perspective view of the generator system of FIGS. 5A and 5B in a clinical environment.
FIG. 5D is a conceptual illustration of various configurations of the generator system of FIGS. 5A and 5B.
FIG. 6 is an isometric view of a generator system not showing all features of the claimed invention.
FIGS. 7A-7I illustrate various remote control devices for use with generator systems configured in accordance with embodiments of the present technology.
FIGS. 8A-8C are a series of screen shots illustrating a generator display configured in accordance with aspects of the present technology.
FIGS. 9A and 9B are screen shots illustrating a generator display configured in accordance with other aspects of the present technology.
FIGS. 10A-10D are a series of screen shots illustrating a generator display configured in accordance with further aspects of the present technology.
FIGS. 11A and 11B are screen shots of a display on a remote control device configured in accordance with aspects of the present technology.
FIG. 12 illustrates the integration of various displays of a generator system configured in accordance with an embodiment of the present technology.
FIG. 13 is a screen shot illustrating a generator display configured in accordance with additional aspects of the present technology.
FIG. 14 is a screen shot illustrating a generator display configured in accordance with further aspects of the present technology.
FIG. 15 is a conceptual illustration of the sympathetic nervous system (SNS) and how the brain communicates with the body via the SNS.
FIG. 16 is an enlarged anatomic view of nerves innervating a left kidney to form the renal plexus surrounding the left renal artery.
FIGS. 17A and 17B are anatomic and conceptual views, respectively, of a human body depicting neural efferent and afferent communication between the brain and kidneys.
FIGS. 18A and 18B are anatomic views of the arterial vasculature and venous vasculature, respectively, of a human.

### DETAILED DESCRIPTION

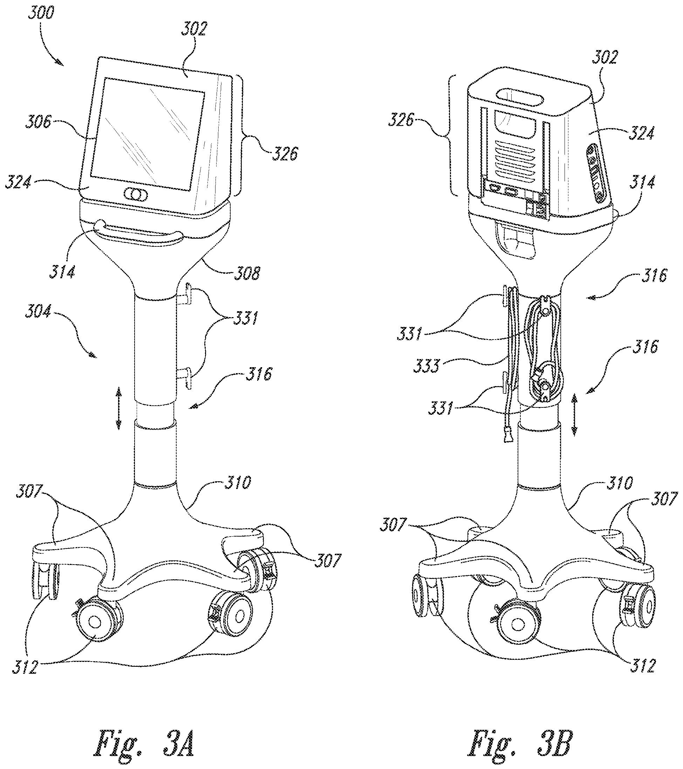

The present invention relates to a generator system as defined claim 1 and further embodied in figures 3A-K.

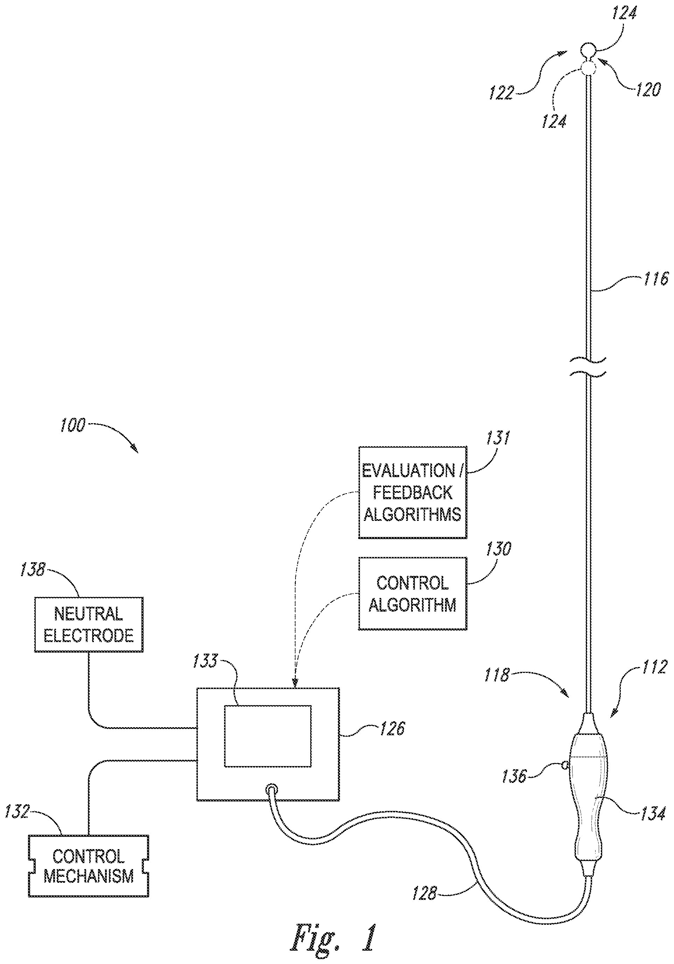

The present technology is generally directed to generator assemblies and systems for renal neuromodulation therapy. In various embodiments, generator assemblies and systems configured in accordance with the present disclosure can also provide feedback relating to operating conditions (e.g., temperature and impedance) during neuromodulation therapies. Specific details of several embodiments of the technology are described below with reference to FIGS. 1-18B. Although many of the embodiments are described below with respect to devices, systems, and methods for providing RF energy for renal neuromodulation, other applications (e.g., providing energy for intravascularly modulating other neural fibers) and other embodiments (e.g., using other forms of electrical energy and/or other types energy) in addition to those described herein are within the scope of the technology. Additionally, several other embodiments of the technology can have different configurations, components, or procedures than those described herein. A person of ordinary skill in the art, therefore, will accordingly understand that the technology can have other embodiments within the scope of independent claim 1.

The terms "distal" and "proximal" are used in the following description with respect to a position or direction relative to the operator or the operator's control device (e.g., a handle assembly). "Distal" or "distally" are a position distant from or in a direction away from the operator or the operator's control device. "Proximal" and "proximally" are a position near or in a direction toward the operator or the operator's control device.

### I. Renal Neuromodulation

Renal neuromodulation is the partial or complete incapacitation or other effective disruption of nerves innervating the kidneys. In particular, renal neuromodulation comprises inhibiting, reducing, and/or blocking neural communication along neural fibers (i.e., efferent and/or afferent nerve fibers) innervating the kidneys. Such incapacitation can be long-term (e.g., permanent or for periods of months, years, or decades) or short-term (e.g., for periods of minutes, hours, days, or weeks). Renal neuromodulation is expected to efficaciously treat several clinical conditions characterized by increased overall sympathetic activity, and in particular conditions associated with central sympathetic over stimulation such as hypertension, heart failure, acute myocardial infarction, metabolic syndrome, insulin resistance, diabetes, left ventricular hypertrophy, chronic and end stage renal disease, inappropriate fluid retention in heart failure, cardio-renal syndrome, osteoporosis, and sudden death. The reduction of afferent neural signals contributes to the systemic reduction of sympathetic tone/drive, and renal neuromodulation is expected to be useful in treating several conditions associated with systemic sympathetic over activity or hyperactivity. Renal neuromodulation can potentially benefit a variety of organs and bodily structures innervated by sympathetic nerves. For example, a reduction in central sympathetic drive may reduce insulin resistance that afflicts patients with metabolic syndrome and Type II diabetics. A more detailed description of pertinent patient anatomy and physiology is provided in Section IV below.

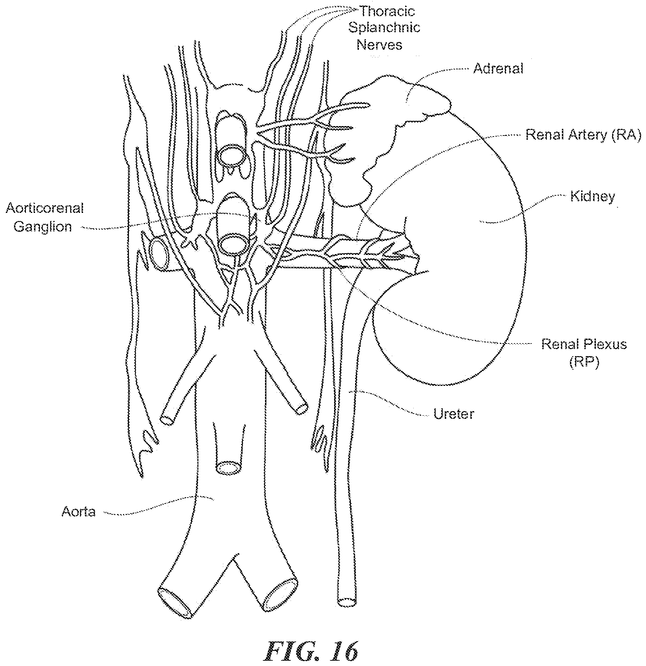

Various techniques can be used to partially or completely incapacitate neural pathways, such as those innervating the kidney. The purposeful application of energy (e.g., electrical energy, thermal energy) to tissue by energy delivery element(s) can induce one or more desired thermal heating effects on localized regions of the renal artery and adjacent regions of the renal plexus (RP) (FIG. 16), which lay intimately within or adjacent to the adventitia of the renal artery. The purposeful application of the thermal heating effects can achieve neuromodulation along all or a portion of the renal plexus (RP).

The thermal heating effects can include both thermal ablation and non-ablative thermal alteration or damage (e.g., via sustained heating and/or resistive heating). Desired thermal heating effects may include raising the temperature of target neural fibers above a desired threshold to achieve non-ablative thermal alteration, or above a higher temperature to achieve ablative thermal alteration. For example, the target temperature can be above body temperature (e.g., approximately 37°C) but less than about 45°C for non-ablative thermal alteration, or the target temperature can be about 45°C or higher for the ablative thermal alteration.

More specifically, exposure to thermal energy (heat) in excess of a body temperature of about 37°C, but below a temperature of about 45°C, may induce thermal alteration via moderate heating of the target neural fibers or of vascular structures that perfuse the target fibers. In cases where vascular structures are affected, the target neural fibers are denied perfusion resulting in necrosis of the neural tissue. For example, this may induce non-ablative thermal alteration in the fibers or structures. Exposure to heat above a temperature of about 45°C, or above about 60°C, may induce thermal alteration via substantial heating of the fibers or structures. For example, such higher temperatures may thermally ablate the target neural fibers or the vascular structures. In some patients, it may be desirable to achieve temperatures that thermally ablate the target neural fibers or the vascular structures, but that are less than about 90°C, or less than about 85°C, or less than about 80°C, and/or less than about 75°C. Regardless of the type of heat exposure utilized to induce the thermal neuromodulation, a reduction in renal sympathetic nerve activity ("RSNA") is expected.

### II. Selected Embodiments of Renal Neuromodulation Systems

FIG. 1 illustrates a neuromodulation system 100 ("system 100") configured in accordance with an embodiment of the present technology. The system 100 includes a treatment device 112 operably coupled to an energy source or energy generator 126 (e.g., an RF energy generator; shown schematically). In the embodiment shown in FIG. 1, the neuromodulation or treatment device 112 (e.g., a catheter) includes an elongated shaft 116 having a proximal portion 118, a handle assembly 134 at a proximal region of the proximal portion 118, and a distal portion 120 extending distally relative to the proximal portion 118. The treatment device 112 further includes a treatment section or therapeutic assembly 122 including an energy delivery element 124 (e.g., an electrode) at or near the distal portion 120 of the shaft 116. In the illustrated embodiment, a second energy delivery element 124 is illustrated in broken lines to indicate that the systems and methods disclosed herein can be used with treatment devices having one or more energy delivery elements 124. Further, it will be appreciated that although only two energy delivery elements 124 are shown, the treatment device 112 may include additional energy delivery elements 124 (e.g., four electrodes, five electrodes, six electrodes, etc.). For example, the therapeutic assembly 122 can be configured to have a helical/spiral shape with a plurality of energy delivery elements 124 positioned thereon. Other helical, spiral and/or multi-electrode therapeutic assemblies 122 may have energy delivery elements 124 with different positions relative to one another than those shown in FIG. 1.

The therapeutic assembly 122 can be delivered intravascularly to a treatment site (e.g., a renal artery) in a low-profile configuration. In one embodiment, for example, the distal end of the therapeutic assembly 122 may define a passageway for engaging a guide wire (not shown) for delivery of the treatment device 112 using over-the-wire ("OTW") or rapid exchange ("RX") techniques. At the treatment site, the therapeutic assembly 122 can transform to a deployed state or arrangement for delivering energy at the treatment site and providing therapeutically-effective electrically-induced and/or thermally-induced neuromodulation. In various embodiments, the therapeutic assembly 122 may be placed or transformed into the deployed state via remote actuation using an actuator 136, such as a knob, pin, or lever carried by the handle assembly 134. In other embodiments, however, the therapeutic assembly 122 may be transformed between the delivery and deployed states using other suitable mechanisms or techniques. In some embodiments, for example, the therapeutic assembly 122 may be delivered to the treatment site within a guide sheath (not shown). When the therapeutic assembly 122 is at the target site, the guide sheath may be at least partially withdrawn or retracted and the therapeutic assembly 122 can move from the low-profile state to the deployed arrangement.

The energy generator 126 (e.g., an RF energy generator) is configured to generate a selected form and magnitude of energy for delivery to the treatment site via the energy delivery element(s) 124. The energy generator 126 can be electrically coupled to the treatment device 112 via a cable 128. At least one supply wire (not shown) can pass along the elongated shaft 116 or through a lumen in the elongated shaft 116 to the energy delivery element(s) 124 and transmit the treatment energy to the energy delivery element(s) 124. As described in greater detail below, a control mechanism, such as a control mechanism 132 (e.g., a foot pedal, a handheld controller, etc.), may be connected (e.g., pneumatically connected, electrically connected, wirelessly connected, etc.) to the energy generator 126 to allow the operator to initiate, terminate and, optionally, adjust various operational characteristics of the energy generator 126 (e.g., power delivery).

The energy generator 126 can be configured to deliver the treatment energy via an automated control algorithm 130 and/or under the control of a clinician. For example, the energy generator 126 can include computing devices (e.g., personal computers, server computers, tablets, etc.) having processing circuitry (e.g., a microprocessor) that is configured to execute stored instructions relating to the control algorithm 130. In addition, the processing circuitry may be configured to execute one or more evaluation/feedback algorithms 131 and may provide feedback to the user (e.g., via a display 133). The display 133 and/or associated features may be configured to provide indications of power levels or sensor data, such as audio, visual and/or other indications, or may be configured to communicate the information to another device. For example, the energy generator 126 may be communicatively coupled to a monitor in a catheterization laboratory. Further details regarding suitable feedback displays, control devices, and associated energy generators are described below with reference to FIGS. 3A-14.

The computing devices associated with the system 100 can further include memory devices, input devices (e.g., a keyboard, mouse, touchscreen, etc.), output devices (e.g., a display device), and storage devices (e.g., disk drives). The output devices may be configured to communicate with the treatment device 112 (e.g., via the cable 128) to control power to the energy delivery element(s) 124 and/or to obtain signals from the energy delivery element(s) 124 or any associated sensors (not shown). The memory and storage devices are computer-readable media that may be encoded with computer-executable instructions that implement the control algorithm 130 and/or evaluation/feedback algorithm(s) 131. The instructions, data structures, and message structures may be stored or transmitted via a data transmission medium, such as a signal on a communications link (e.g., the Internet, a local area network, a wide area network, a point-to-point dial-up connection, a cell phone network, etc.).

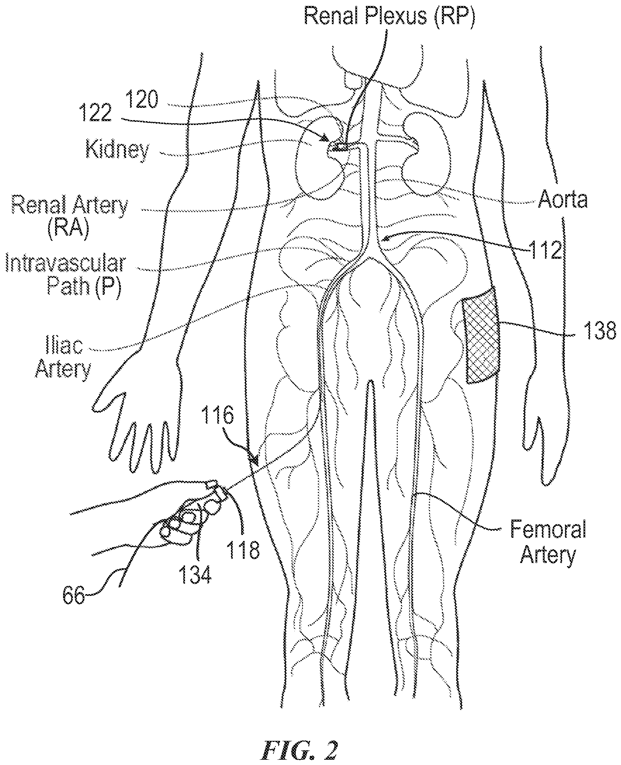

In selected embodiments, the system 100 may be configured to provide delivery of a monopolar electric field via the energy delivery element 124. In such embodiments, a neutral or dispersive electrode 138 (FIG. 2) may be electrically connected to the energy generator 126 and attached to the exterior of the patient. Additionally, one or more sensors (not shown), such as one or more temperature (e.g., thermocouple, thermistor, etc.), impedance, pressure, optical, flow, chemical and/or other sensors, may be located proximate to or within the energy delivery element 124. The sensor(s) and the energy delivery element 124 can be connected to one or more supply wires (not shown) that transmit signals from the sensor(s) and/or convey energy to the energy delivery element(s) 124.

In embodiments including multiple energy delivery elements 124, the energy delivery elements 124 may deliver power independently (i.e., may be used in a monopolar fashion), either simultaneously, selectively, or sequentially, and/or may deliver power between any desired combination of the energy delivery elements 124 (i.e., may be used in a bipolar fashion). Furthermore, the operator optionally may be permitted to choose which energy delivery element(s) 124 are used for power delivery in order to form customized lesion(s) within the renal artery, as desired.

FIG. 2 (with additional reference to FIG. 1) illustrates modulating renal nerves with an embodiment of the system 100. The treatment device 112 provides access to the renal plexus RP through an intravascular path P, such as a percutaneous access site in the femoral (illustrated), brachial, radial, or axillary artery to a targeted treatment site within a respective renal artery RA. As illustrated, a section of the proximal portion 118 of the shaft 116 is exposed externally of the patient. By manipulating the proximal portion 118 of the shaft 116 from outside the intravascular path P, the clinician may advance the shaft 116 through the sometimes tortuous intravascular path P and remotely manipulate the distal portion 120 of the shaft 116. In the embodiment illustrated in FIG. 2, the therapeutic assembly 122 is delivered intravascularly to the treatment site using a guide wire 66 in an OTW technique. As noted previously, the distal end of the therapeutic assembly 122 may define a passageway for receiving the guide wire 66 for delivery of the treatment device 112 using either OTW or RX techniques. At the treatment site, the guide wire 66 can be at least partially withdrawn or removed, and the therapeutic assembly 122 can transform or otherwise be moved to a deployed arrangement for delivering energy at the treatment site. In other embodiments, the therapeutic assembly 122 may be delivered to the treatment site within a guide sheath (not shown) with or without using the guide wire 66. When the therapeutic assembly 122 is at the target site, the guide sheath may be at least partially withdrawn or retracted and the therapeutic assembly 122 can be transformed into the deployed arrangement. In still other embodiments, the shaft 116 may be steerable itself such that the therapeutic assembly 122 may be delivered to the treatment site without the aid of the guide wire 66 and/or guide sheath.

Image guidance, e.g., computed tomography (CT), fluoroscopy, intravascular ultrasound (IVUS), optical coherence tomography (OCT), intracardiac echocardiography (ICE), or another suitable guidance modality, or combinations thereof, may be used to aid the clinician's positioning and manipulation of the therapeutic assembly 122. For example, a fluoroscopy system (e.g., including a flat-panel detector, x-ray, or c-arm) can be rotated to accurately visualize and identify the target treatment site. In other embodiments, the treatment site can be determined using IVUS, OCT, and/or other suitable image mapping modalities that can correlate the target treatment site with an identifiable anatomical structure (e.g., a spinal feature) and/or a radiopaque ruler (e.g., positioned under or on the patient) before delivering the treatment device 112. Further, in some embodiments, image guidance components (e.g., IVUS, OCT) may be integrated with the treatment device 112 and/or run in parallel with the treatment device 112 to provide image guidance during positioning of the therapeutic assembly 122. For example, image guidance components (e.g., IVUS or OCT) can be coupled to the therapeutic assembly 122 to provide three-dimensional images of the vasculature proximate the target site to facilitate positioning or deploying the multi-electrode assembly within the target renal blood vessel.

The purposeful application of energy from the energy delivery elements 124 (FIG. 1) may then be applied to target tissue to induce one or more desired neuromodulating effects on localized regions of the renal artery and adjacent regions of the renal plexus RP, which lay intimately within, adjacent to, or in close proximity to the adventitia of the renal artery RA. The purposeful application of the energy may achieve neuromodulation along all or at least a portion of the renal plexus RP. The neuromodulating effects are generally a function of, at least in part, power, time, contact between the energy delivery elements 124 (FIG. 1) and the vessel wall, and blood flow through the vessel. The neuromodulating effects may include denervation, thermal ablation, and/or non-ablative thermal alteration or damage (e.g., via sustained heating and/or resistive heating). Desired thermal heating effects may include raising the temperature of target neural fibers above a desired threshold to achieve non-ablative thermal alteration, or above a higher temperature to achieve ablative thermal alteration. For example, the target temperature may be above body temperature (e.g., approximately 37°C) but less than about 45°C for non-ablative thermal alteration, or the target temperature may be about 45°C or higher for the ablative thermal alteration. Desired non-thermal neuromodulation effects may include altering the electrical signals transmitted in a nerve.

### III. Generator Assemblies and Systems

A variety of different generator systems and related components for use with suitable neuromodulation systems (e.g., the neuromodulation system of FIGS. 1 and 2) are described below with reference to FIGS. 3A-14. The generator systems and related components can serve as an energy source (e.g., the generator 126 of FIG. 1) for the neuromodulation systems and provide feedback related to the treatment sessions. It will be appreciated that the generator systems described below and/or specific features thereof can be used with the neuromodulation system components described above (e.g., the treatment device 112 shown in FIGS. 1 and 2), used with other suitable neuromodulation system components, and/or used as standalone or self-contained devices.

FIGS. 3A and 3B are front and back isometric views, respectively, of a generator system 300 ("system 300") configured in accordance with an embodiment of the present invention. The system 300 can include an energy source or generator assembly 302 and includes a display 306 carried by a base, cart, or stand assembly 304. The generator assembly 302 is configured to convey energy to a neuromodulation treatment device (e.g., the treatment device 112 shown in FIG. 1). In various embodiments, for example, the generator assembly 302 can provide one or more energy channels (e.g., RF energy channels) to a single or multi-electrode treatment device. In one particular embodiment, for example, the generator assembly 302 can include four RF energy channels to selectively provide energy to a four-electrode treatment device and/or treatment devices having a different number of electrodes (i.e., more or fewer than four electrodes). The display 306 can be configured to communicate information related to neuromodulation therapies, such as the operating conditions (e.g., impedance, temperature, etc.) of the treatment device and/or the generator assembly 302.

The stand assembly 304 can include a first or body portion 308 configured to carry the generator assembly 302 and the display 306 and a second or base portion 310 configured to provide a stable base structure for the system 300. In some embodiments, the base portion 310 can include one or more wheels 312 and the body portion 308 can include a grip or handle 314 to facilitate transportation of the system 300. In the illustrated embodiment, for example, the base portion 310 includes five wheels 312 attached to five corresponding arm members 307. The arm members 307 can extend radially outward from a central axis of the base portion 310 (e.g., in a star-shaped or flower pedal pattern) to provide a stable structure with which to support the generator assembly 302. In other embodiments, the base portion 310 can include fewer than or more than five wheels 312 attached to a corresponding number of arm members 307, or the arm members 307 can each have more than one wheel 312 attached thereto. In further embodiments, the base portion 310 can have other suitable configurations that stabilize the stand assembly 304 and facilitate transportation thereof In various embodiments, the stand assembly 304 can include an adjustable member 316 that allows users to change the height of the system 300 (e.g., by approximately 30 cm (11.8 in)). As such, the system 300 can be raised (e.g., to accommodate standing use) and lowered (e.g., to accommodate seated use and/or storage). In other embodiments, the member 316 is not height adjustable (e.g., a solid or continuous pole or rod). The stand assembly 304 can also include one or more cable holders 331 (e.g., hooks) positioned along a portion of the stand assembly 304 and configured to retain cords 333 associated with the generator assembly 302 and/or the display 306 (e.g., power cords, video connector cables, etc.). In other embodiments, the stand assembly 304 can include other storage features on or in the body portion 308 and/or base portion 310 to compactly retain the cords 333. In further embodiments, the stand assembly 304 can include additional storage features for holding or retaining other components associated with the system 300 (e.g., a neuromodulation catheter, remote controls, etc.).

The display 306 can include a screen or monitor that has a suitable resolution and size to illustrate various operating conditions of the system 300 and/or other related information. For example, the display 306 can be approximately 9-12 inches (22.9-30.5 cm) and include a digital visual interface ("DVI"), a visual graphics array ("VGA"), a high-definition multimedia interface ("HDMI"), and/or other high resolution displays. In other embodiments, the display 306 can be larger or smaller and/or be coupled to other types of displays or indicators (e.g., audible indicators, LED indicators, etc.) The display 306 can also be configured as a touchscreen that serves as a user interface for controlling the system 300 and/or otherwise interacting with the operator.

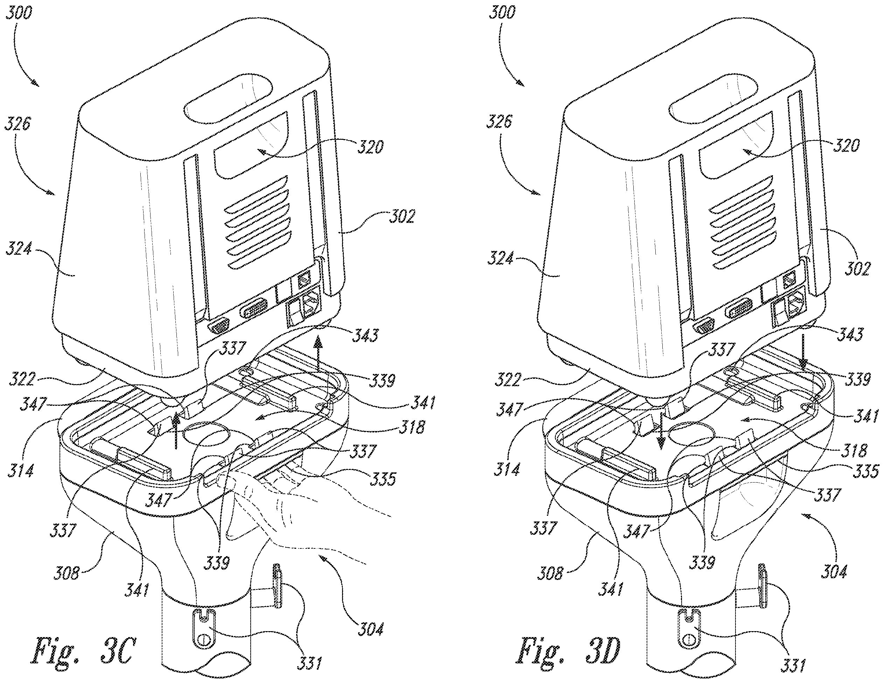

In the embodiment illustrated in FIGS. 3A and 3B, the generator assembly 302 and the display 306 are integrated into a single housing 324 to define a generator console 326 ("console 326"). The console 326 can serve as a compact standalone energy source that provides RF or other forms of energy via the generator assembly 302 and communicates operating conditions of neuromodulation therapies via the display 306. FIGS. 3C and 3D, for example, are back isometric views of the console 326 as it is being separated from and attached to the stand assembly 304, respectively. The stand assembly 304 includes a recessed portion 318 configured to receive a corresponding portion, e.g., a pedestal or base portion 322, of the console 326. The recessed portion 318 includes locking members 337 (e.g., latches, clasps, hooks, etc.) that interface with corresponding portions of the console 326 to removably connect the console 326 to the stand assembly 304. The locking members 337 are spring loaded such that the weight of the console 326 on the locking members 337 can automatically latch the locking members 337 to the console 326 as the console 326 is placed onto the stand assembly 304. The recessed portion 318 can also include a plurality of protrusions 341 and/or grooves 343 that interface or mate with corresponding recesses or protrusions on the underside of the console 326 to enhance the connection between the console 326 and the stand assembly 304. The protrusions 341 and/or grooves 343 can also aid in positioning (e.g., aligning or keying) the console 326 with respect to the recessed portion 318 to facilitate proper attachment of the locking members 337 to the console 326.

As further shown in FIGS. 3C and 3D, the body portion 308 of the stand assembly 304 includes a release or disengagement mechanism 335 (e.g., a handle, lever, button, etc.) that a user can actuate (e.g., by squeezing, compressing, twisting, or otherwise manipulating the release mechanism 335) to move the locking members 337 from a locked arrangement (FIG. 3D) to an unlocked arrangement (FIG. 3C), and free the console 326 from the stand assembly 304. As shown in FIG. 3C, for example, compressing the release mechanism 335 can move the locking members 337 rearward within corresponding openings 339 in the recessed portion 318 of the stand assembly 304 to disengage the locking members 337 from the console 326. This detaches the console 326 from the stand assembly 304, and allows the user to remove the console 326 from the stand assembly 304. In the illustrated embodiment, the individual locking members 337 can have a wedge-like shape (i.e., a generally triangular cross-section) protruding away from the recessed portion 318, and a hook 347 at the end of each wedge-shaped locking member 337 that can latch to or otherwise engage a portion of the console 326. In other embodiments, the locking members 337 can have other suitable shapes and/or engagement features for connecting to the console 326. In various embodiments, the console 326 can include a grip or handle portion 320 to facilitate removing the console 326 from the stand assembly 304 and ease subsequent transport of the console 326.

As shown in FIG. 3D, to re-attach the console 326, the user can lower the console 326 into the recessed portion 318 of the stand assembly 304 and, optionally, use the corresponding protrusions 341 and grooves 343 to align the console 326 therein. The weight of the console 326 on the locking members 337 automatically latches or otherwise couples the locking members 337 to the corresponding portions of the console 326, and thereby releasably attach the two components together. In various embodiments, the attachment of the locking members 337 to the console 326 can emit an audible sound (e.g., a click) to confirm that a proper connection has been made. In other embodiments, the release mechanism 335 can be manipulated (e.g., compressed) to retract or otherwise move the locking members 337 as the console 326 is positioned within the recessed portion 318, and released to move the locking members 337 into the locking arrangement to connect with the console 326. In further embodiments, the console 326 can be removably attached to the stand assembly 304 using other suitable fastening methods known in the art (e.g., an interference fit between mating members).

The portable console 326 has a relatively small footprint, and can therefore be positioned virtually anywhere in a catheterization laboratory or other suitable location for neuromodulation therapy. For example, the console 326 can rest on an equipment bench proximate a patient table or on the patient table itself. The console 326 may also be mounted to another structure, such as a bed rail or an IV pole (e.g., using a VESA mount). In other embodiments, however, the display 306 and the generator assembly 302 can be separate components that are spaced apart from one another. For example, the generator assembly 302 and the display 306 can be positioned on different portions of the stand assembly 304, or the display 306 may be spaced apart from the stand assembly 304 in a remote location (e.g., proximate to other monitors in a catheterization laboratory and/or integrated with the monitors in the catheterization laboratory) and communicatively coupled to the generator assembly 302. Further details regarding such arrangements are described below.

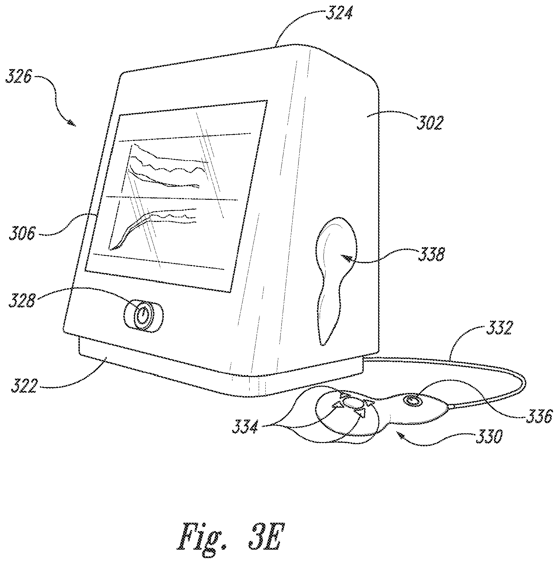

FIGS. 3E-3G illustrate various isometric views of the console 326. Referring first to FIG. 3E, the console 326 can include a power button 328 configured to activate the generator assembly 302 or the console 326 as a whole. The console 326 can also include a user interface configured to control the application of energy to a treatment site (e.g., an inner wall of a renal artery) via a neuromodulation device. In the illustrated embodiment, the user interface includes a remote control device 330 that is operatively coupled to the generator assembly 302 via a cable 332. The remote control device 330 can be durable (e.g., non-disposable) or disposable, and can be wirelessly coupled to the generator assembly 302. The remote control device 330 can include an energy activation button 336 that initiates delivery of RF or other forms of energy to the treatment site and a plurality of command or selector buttons 334 used to modulate energy delivery. As shown in FIG. 3E, the selector buttons 334 can be arranged in a circular pattern that distinguishes the buttons 334 from one another such that the operator can control energy delivery without having to look at the remote control device 330.

In various embodiments, each selector button 334 can correspond to an energy channel (e.g., an RF energy channel) of the generator assembly 302, and can be used to select and/or deselect corresponding energy delivery elements (e.g., RF electrodes) of a neuromodulation catheter (e.g., the treatment device 112 of FIG. 1) communicatively coupled to the remote control device 330. Accordingly, the remote control device 330 allows a physician to independently control the energy channels to selectively deliver energy to a treatment site (e.g., an arterial wall) and produce a desired ablation pattern. In some embodiments, the selector buttons 334 can be backlit or otherwise illuminated with one or more colors (e.g., colored LEDs) that relate to the operating condition of the corresponding energy channel. For example, each selector button 334 can be independently illuminated with a first color (e.g., green) when the corresponding energy channel is selected (e.g., when energy is being delivered to the treatment site), and illuminated with a second color (e.g., amber) different from the first color when the operating condition of the corresponding energy channel changes in a predetermined manner (e.g., falls outside a desired temperature range, impedance range, power range, etc.). The selector buttons 334 can be unilluminated when the corresponding energy delivery channels are deselected. Similarly, the activation button 336 can be backlit or otherwise illuminated (e.g., using a blue LED) when generator assembly 302 has been activated (e.g., ready to delivery energy via the energy channels), and not backlit when energy delivery has not been activated. In certain embodiments, the button lighting scheme of the remote control device 330 can sync with the operating condition display shown on the touchscreen display 306 or other associated user interface. For example, a user can activate the energy channels via the touchscreen display 306 or the remote control device 330, and the button lighting/operating condition display on the other device (i.e., the display 306 or the remote control device 330) can sync with that of the device used to activate the energy channels.

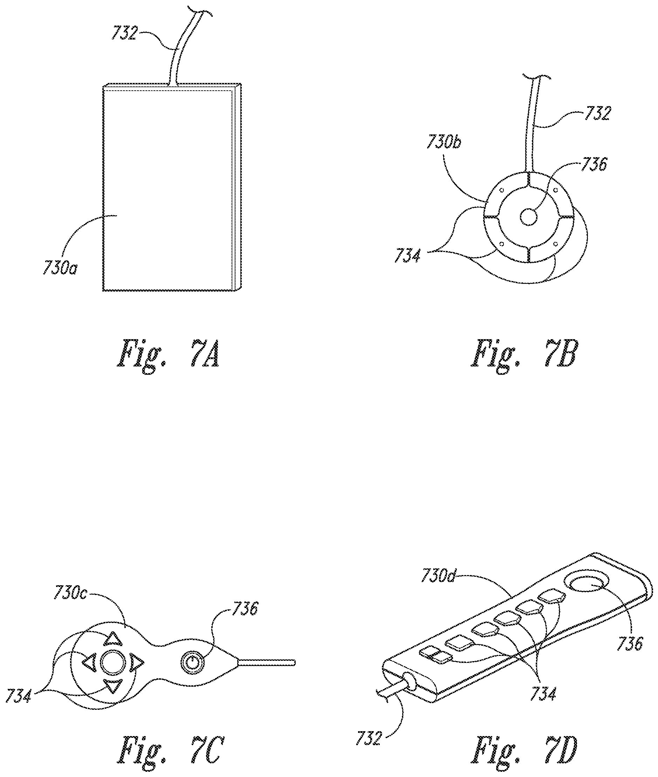

In other embodiments, the remote control device 330 can include additional command buttons, some of the buttons 334, 336 can be omitted, and/or the buttons 334, 336 can have other suitable configurations. For example, the remote control device 330 can include scroll buttons that allow a user to navigate and/or otherwise control a user interface (e.g., the touchscreen display 306). Additional embodiments of suitable remote control devices for use with generator consoles (e.g., the generator console 326) are described in further detail below with reference to FIGS. 7A-7I. In various embodiments, the console 326 can be configured to retain the remote control device 330 for compact storage. For example, in the embodiment illustrated in FIG. 3E, the console 326 includes a recessed portion 338 of the housing 324 configured to receive the remote control device 330.

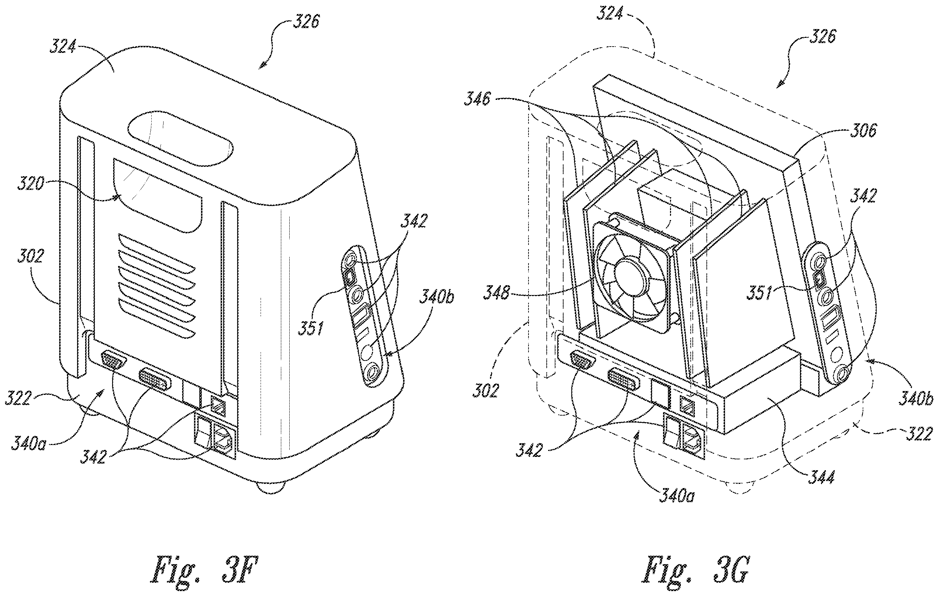

As shown in FIG. 3F, the console 326 can further include a plurality of cable connection interfaces, such as a first cable interface 340a associated with the generator assembly 302 and a second cable interface 340b associated with the display 306 (FIG. 3E). The first and second cable interfaces 340a, 340b (referred to collectively as the cable interfaces 340) can include one or more adaptors or ports 342 configured connect the generator assembly 302, the display 306 (FIG. 3E), and/or other features of the console 326 with various peripheral devices. For example, the first cable interface 340a can include one or more ports for a neuromodulation device, a port for a return electrode (e.g., configured to convey sensed data from the neuromodulation device), a port for a neutral or dispersive electrode (e.g., the dispersive electrode 138 of FIG. 2), a USB port, an AC power supply port, a network connection port, and/or other suitable ports. The second cable interface 340b can include various video and audio ports related to the display 306. In the illustrated embodiment, the first cable interface 340a is accessible through an opening in the housing 324 at the back of the console 326, and the second cable interface 340b is exposed through an opening at the side of the console 326. In other embodiments, however, the cable interfaces 340 can be located elsewhere on the console 326, the cable interfaces 340 can be combined into a single cable interface, and/or the console 326 can include additional cable interfaces. In further embodiments, the two cable interfaces 340 can include identical ports 342, therefore allowing the operator to select which location of the cable interfaces 340 (e.g., the back or the side) is more convenient to use.

FIG. 3G illustrates selected internal features of the console 326, such as the generator assembly 302. In the illustrated embodiment, the generator assembly 302 includes a power supply 344, a fan 348, and four RF modules or boards 346 configured to convey RF energy through four channels that correspond with one or more electrodes of a neuromodulation device. In other embodiments, the generator assembly 302 can include one, two, three, or more than four RF boards 346 and/or be configured to generate other forms of energy. In further embodiments, the console 326 can include additional features that facilitate energy generation and/or provide monitoring and feedback related to energy delivery.

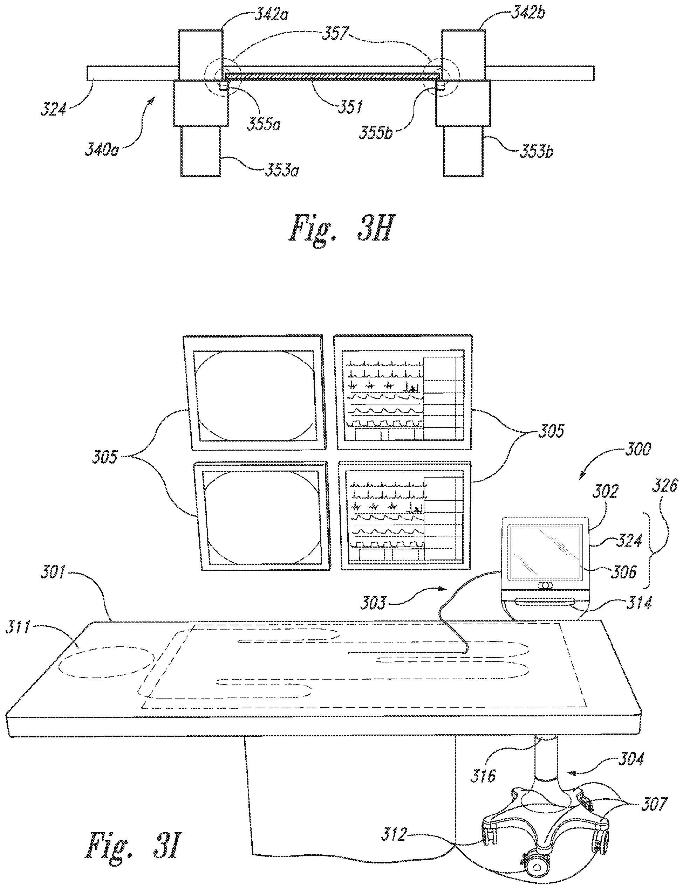

As further shown in FIGS. 3F and 3G, the console 326 can include a radiofrequency identification (RFID) reader-writer module 351 ("RFID module 351") mounted at or near one or more connector ports 342 and configured to wirelessly read and write to RFID tags on devices (e.g., a neuromodulation catheter) removably coupled to the generator assembly 302 via the ports 342. FIG. 3H, for example, is a schematic top view of a plurality of neuromodulation catheters (identified individually as a first catheter 353a and a second catheter 353b, and referred to collectively as catheters 353) attached to corresponding first and second ports 342a and 342b of the first connector interface 340a. The first and second catheters 353a and 353b can each include an RFID tag (identified individually as a first RFID tag 355a and a second RFID tag 355b, and referred to collectively as RFID tags 355) housed within or otherwise attached to the connector portions of the catheters 353. The RFID tags 355 can include an antenna and an RFID chip for processing signals, sending/receiving RF signals, and storing data in memory. Suitable RFID tags include, for example, MB89R118 RFID tags commercially available from Fujitsu Ltd. of Tokyo, Japan. The memory portion of the RFID tags 355 can include a plurality of blocks allocated for different types of data. For example, a first memory block can include a validation identifier (e.g., a unique identifier associated with the specific type of catheter and generated from the unique ID of the RFID tag 355 using an encrypting algorithm), and a second memory block can be allocated as a catheter usage counter that can be read and then written to by the RFID module 351 after catheter use. In other embodiments, the RFID tags 355 can include additional memory blocks allocated for additional catheter usage counters (e.g., to allow the catheter 353 to be used a specific limited number of times) and/or other information associated with the catheter 353 (e.g., lot number, customer number, catheter model, summary data, etc.).

The RFID module 351 can include an antenna and a processing circuit that are together used to communicate with the generator assembly 302 (e.g., a generator controller) and wirelessly read and write to one or more RFID tags 355 within its proximity. As shown in FIG. 3H, for example, the RFID module 351 can have a communication field 357 (shown in broken lines) within which the RFID module 351 can communicate with the RFID tags 355 of the catheters 353 when the catheters 353 are attached to the ports 342. Suitable RFID reader-writer modules include, for example, a TRF7960A Evaluation Module commercially available from Texas Instruments, Inc. of Dallas, Texas.

Referring to FIGS. 3F-3H together, the RFID module 351 can read information from the RFID tags 355, and communicate the information to generator software to validate the attached catheters 353 (e.g., validate that the catheter model is compatible with the generator assembly 302), read the number of previous uses associated with each catheter 353, and/or write to the RFID tags 355 to indicate catheter use. In various embodiments, the generator assembly 302 (e.g., via generator software) can be configured to disable energy delivery to the catheter(s) 353 when predefined conditions of the RFID tags 355 are not met. For example, when each catheter 353 is connected to the generator assembly 302 (e.g., via the port 342), the RFID module 351 can read a unique anti-counterfeit number in an encrypted format from the RFID tag 355, decrypt the number, and then authenticate the number and the catheter data format for recognized catheters (e.g., catheters that are compatible with the generator assembly 302, non-counterfeit catheters, etc.). In various embodiments, the RFID tags 355 can include identifiers that correspond to a specific type of catheter 353, and the RFID module 351 can transmit this information to a main generator controller, which adjusts the settings of the generator assembly 302 to the operating parameters/characteristics (e.g., power levels, display modes, etc.) associated with the specific catheter. If the RFID module 351 identifies the catheter 353 as counterfeit or is otherwise unable to identify the catheter 353, the generator assembly 302 can automatically disable the use of the catheter 353 (e.g., preclude energy delivery).

Once the catheter 353 has been identified, the RFID module 351 can read the RFID tag memory address spaces to determine if the catheter 353 was previously connected to a generator (i.e., previously used). In certain embodiments, the RFID tag 355 will limit the catheter 353 to a single use, but in other embodiments the RFID tag 355 can be configured to provide for more than one use (e.g., 2 uses, 5 uses, 10 uses, etc.). If the RFID module 351 recognizes that the catheter 353 has been written (i.e., used) more than a predetermined use limit, the RFID module 351 will communicate with the generator assembly 302 to disable energy delivery to the catheter 353. In certain embodiments, the RFID module 351 can be configured to interpret all the catheter connections to a generator system within a predefined time period (e.g., 5 hrs, 10 hrs, 24 hours, 30 hours, etc.) as single connection (i.e., a single use), and allow the catheter 353 to be used multiple times within the predefined time period. After the catheter 353 has been detected, recognized, and judged as a "new connection" (e.g., not used more than the predefined limit), the RFID module 351 can write to the RFID tag 355 the time and date of the system use and/or other information to indicate that the catheter 353 has been used.

FIG. 3I is a perspective view of the system 300 in a clinical environment (e.g., a catheterization laboratory) configured in accordance with an embodiment of the present technology. The system 300 has a relatively small footprint, and can therefore be positioned in various locations in the clinical setting, such as proximate to a patient 311 (shown schematically in broken lines). For example, in the illustrated embodiment, the system 300 is positioned behind a patient table 301 and the display 306 is substantially aligned with other screens or monitors 305 (e.g., fluoroscopy screens for image guidance) by manipulating the adjustable member 316. In other embodiments, the console 326 can be disconnected from the stand assembly 304 (e.g., as shown in FIGS. 3C and 3D) and configured to rest on the patient table 301 or be mounted to a nearby structure (e.g., an IV pole or bed rail), thereby further minimizing the space required for the console 326 in the clinical environment.

During a neuromodulation procedure, locations near the patient are typically within a sterile field. In the embodiment illustrated in FIG. 3I, the console 326 and the stand assembly 304 are positioned outside the sterile field A neuromodulation device 303 is coupled to the generator assembly 302 (e.g., via the first connection interface 340a shown in FIGS. 3F and 3G) such that it extends from the console 326 into the sterile field where it can be navigated through the vasculature of a patient to a target site (e.g., a renal artery). Once at the target site, an operator can convey energy (e.g., RF energy) to electrodes at the distal portion of the neuromodulation device 303 and control the application thereof using the remote control device 330 (FIG. 3E). In certain embodiments, the remote control device 330 (FIG. 3E) may be placed in a sterile covering (e.g., a plastic bag) for use within the sterile field. In other embodiments, the system 300 can include other user interfaces (e.g., a touchscreen, foot pedal, disposable sterile remotes, etc.) for controlling energy delivery. In further embodiments, the system 300 and/or portions thereof can be configured for use within the sterile field. For example, rather than sterilizing the system 300, a sterile barrier (e.g., a bag) can be provided around portion of the system 300.

As mentioned above, the display 306 can be configured to show operating characteristics/parameters of the neuromodulation device 303 and the generator assembly 302 before, during, and/or after energy delivery. For example, the display 306 can be configured to plot the impedance of one or more electrodes at the distal portion of the neuromodulation device in real time to assist the operator in determining whether sufficient contact has been made between the electrodes and tissue at the target site. Such impedance plots can also be used to indicate the status of each electrode (e.g., whether the electrode is in a stable position and in good contact with tissue at the target site, whether the electrode has moved out of contact with the tissue, etc.). For example, a change (e.g., a decrease) in impedance is thought to indicate instability in the corresponding electrode's contact with the tissue. The display 306 may also or alternatively be configured to indicate the temperature at each electrode. This information can be used to identify various characteristics/parameters associated with the electrodes and/or aspects of the neuromodulation therapy. For example, temperature measurements can be used to determine electrode contact and/or electrode stability, as a safety precaution (e.g., to reduce the likelihood of exposing tissue to temperatures above a predetermined threshold), and/or other characteristics derived from temperature measurements. In other embodiments, the display 306 can also be configured to indicate other operational characteristics/parameters (e.g., power, ranges of impedance or temperature based on standard deviation or threshold values, etc.) that can provide information to the user regarding electrode contact with a vessel wall and/or other information related to neuromodulation therapies. In various embodiments, the system 300 can be coupled to the remote monitors 305, and these additional screens can be selectively used to view the operational characteristics of the system 300 (e.g., to view additional information or to display the same information as display 306 on a larger screen). Upon completion of the neuromodulation therapy, the system 300 can be placed in a compact configuration (e.g., by changing the height via the adjustable member 316) for convenient storage, or the console 326 can be stored separate from the stand assembly 304 on a shelf or in a cabinet.

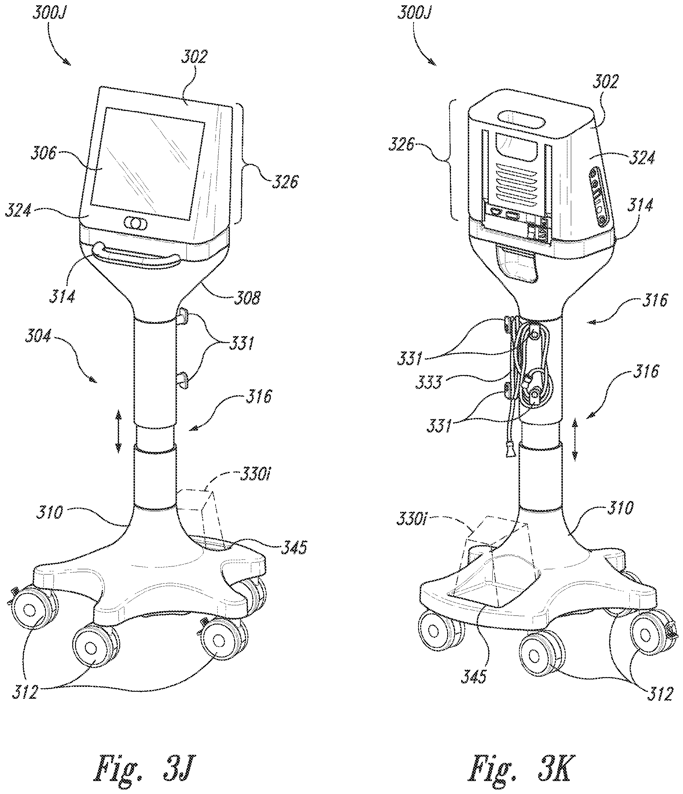

FIGS. 3J and 3K are front and back isometric views, respectively, of a generator system 300J ("system 300J") configured in accordance with another embodiment of the present technology. The system 300J includes various features generally similar to the features of the system 300 described above with reference to FIGS. 3A-3I. For example, the system 300J includes the cart assembly 304 and the generator console 326, which includes the generator assembly 302 and the display 306. In this embodiment, however, the base portion 310 of the cart assembly 304 includes a foot pedal holder 345 for retaining a foot pedal 330i (shown in broken lines), which can be operably coupled to the generator assembly 302 and used to activate and/or otherwise control energy delivery from the generator assembly 302. The foot pedal holder 345, therefore, allows the foot pedal 330i to be moved together with the generator console 302, and facilitates transport of the system 300J as a whole. In the embodiment illustrated in FIGS. 3J and 3K, the foot pedal holder 345 is defined by an indented or recessed section of the base portion 310 shaped to receive the foot pedal 330i. In other embodiments, the foot pedal holder 345 can include additional fasteners for securing the foot pedal 330i to the cart assembly 304, and/or be positioning on other portions of the cart assembly 304 that allow for compact transportation of the system 300J.

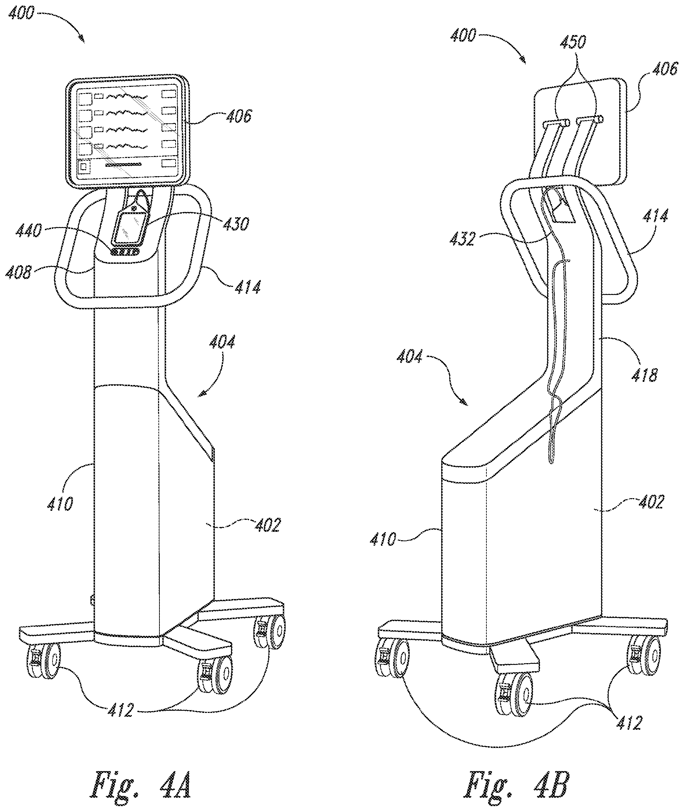

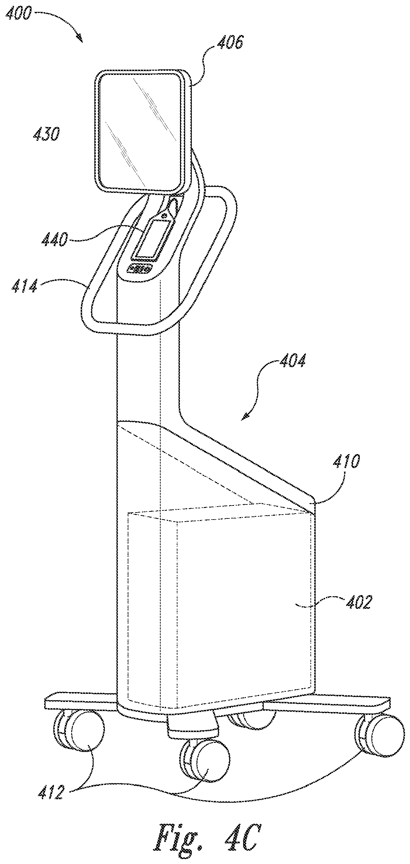

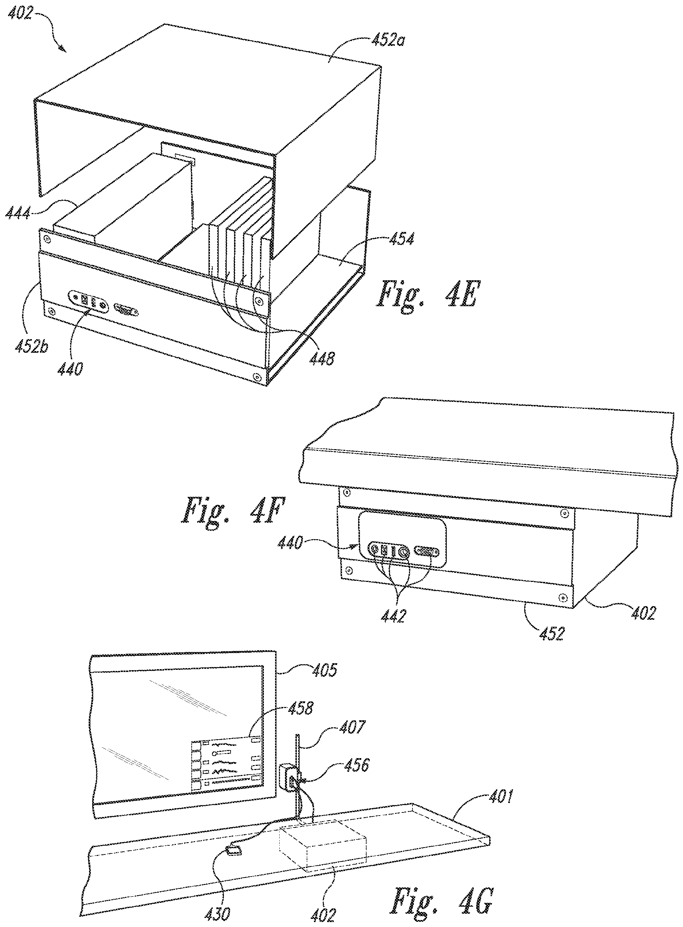

FIGS. 4A-4C are isometric views of a generator system 400 ("system 400") not showing all features of the claimed invention. The system 400 can include features generally similar to the systems 300 and 300J described above with reference to FIGS. 3A-3K. For example, the system 400 can include a generator assembly 402 (e.g., an RF generator) and a display 406 carried by a stand assembly 404. In this embodiment, however, the generator assembly 402 and the display 406 are not integrated into a single console, but are separate components spaced apart from one another on the stand assembly 404. As shown in FIGS. 4A-4C, for example, the generator assembly 402 can be housed in a base portion 410 of the stand assembly 404 (e.g., in a sheet metal shroud; FIG. 4C), and the display 406 is mounted on supports 450 (FIG. 4B) extending from a body portion 408 of the stand assembly 404. The generator assembly 402 and the display 406 can be operably coupled together using a wireless connection and/or electrical connectors (not shown) extending through the body of the stand assembly 404. As described in further detail below with reference to FIGS. 4E-4G, the generator assembly 402 may be removed from the stand assembly 404, and attached to a separate shelf system (e.g., using a VESA mount), positioned under a patient table, and/or integrated with other aspects of a catheterization laboratory. In various embodiments, the supports 450 can be configured to swivel, tilt, and/or adjust the height of the display 406 to change the viewing angle of the display 406 (e.g., for operators of different heights) and/or reduce glare. A handle 414 can extend around the body portion 408 of the stand assembly 404 to provide a barrier around the system 400 that reduces the likelihood of damage to the display 406, and also aids in maneuvering the stand assembly 404.

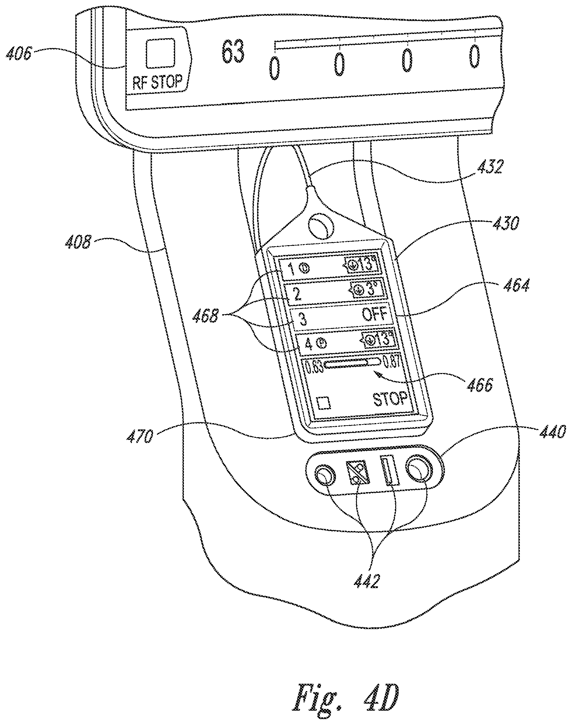

As shown in FIGS 4A and 4C, a cable interface 440 can be spaced apart from and operably coupled to the generator assembly 402 such that the cable interface 440 is positioned at an easily accessible location on the body portion 408 of the stand assembly 404. FIG. 4D is an enlarged view of the cable interface 440 on the body portion 408 of the stand assembly 404. The ports 442 can be configured to connect the generator assembly 402 with, e.g., a neuromodulation device, a return electrode, and/or other suitable peripheral devices. In other embodiments, the system 400 can include additional cable interfaces located elsewhere on the system 400. For example, a cable interface on the generator assembly 402 may be accessed via an opening in the housing of the base portion 410.

As further shown in FIG. 4D, the body portion 408 of the stand assembly 404 can also include a holder or recess 470 configured to retain a remote control device 430. Similar to the remote control device 330 described above with reference to FIG. 3D, the remote control device 430 in the illustrated embodiment is a handheld device that is hardwired to the generator device 402 via a cable 432. However, rather than physical command buttons, the remote control device 430 includes a touchscreen 464 that receives commands (e.g., to control energy delivery) via finger taps. As will be described in greater detail below, the touchscreen 464 can also be configured to provide visual indicators related to the operating conditions of the system 400 and the neuromodulation device. For example, in the illustrated embodiment, the touchscreen 464 includes a timer 466 and a plurality of RF channel indicators 468 that provide operational characteristics (e.g., temperature, impedance, activation status, etc.) corresponding to one or more electrodes on a neuromodulation device. In other embodiments, the touchscreen 464 can display other information related to the system 400. In further embodiments, the remote control device 430 can also include physical control buttons to supplement the touchscreen 464.

Referring to FIGS. 4C and 4D together, the system 400 can include a plurality of wheels 412 at the base portion 410 of the stand assembly 404, such that the stand assembly 404 can be rolled to a desired location within a clinical environment, and locked to secure the system 400 in place (e.g., during neuromodulation therapy). A neuromodulation device (not shown) and other peripheral devices (e.g., display screens) can be operatively coupled to the system 400 via the easily accessible cable interface 440. Once the distal portion of the neuromodulation device is positioned at a treatment site (e.g., a renal artery) within a patient, the remote control device 430 can be used to initiate and control the application of energy (e.g., RF energy) from the generator assembly 402 to the treatment site. The remote control device 430 can remain mounted on the holder 470 during the treatment for easy viewing, or the remote control device 430 can be removed from the holder 470, (e.g., enclosed in a sterile barrier for use by the treating clinician). In various embodiments, the display 406 can be configured as a touchscreen to serve as an additional or alternative control mechanism. For example, the display 406 may be used to control aspects of the system 400 that benefit from a larger visual display (e.g., than the touchscreen 464 on the remote control device 430). Before, during, and/or after the application of energy to the treatment site, the display 406 and/or the touchscreen 464 on the remote control device 430 can provide real time information related to the operating conditions of the system 400 and the neuromodulation device. Once the procedure is complete, the system 400 can conveniently be rolled into a storage space for later use.

In various embodiments, the system 400 can be configured to allow the generator assembly 402 to be used as a standalone device independent of the stand assembly 404. For example, FIGS. 4E and 4F are exploded isometric and isometric views, respectively, of the generator assembly 402 of FIGS. 4A-4C after being removed from the stand assembly 404. The generator assembly 402 can include features generally similar to the features of the generator assembly 302 described above with reference to FIGS. 3A-3G. As shown in FIG. 4E, for example, the generator assembly 402 includes a power supply 444 and a plurality of RF daughter boards 448 coupled to a printed circuit board assembly ("PCBA") 454. A housing 452 (shown in FIG. 4E as a first housing portion 452a and a second housing portion 452b) can be made from a durable material, such as a sheet metal or milled aluminum, to enclose and protect the internal components of the generator assembly 402. A cable interface 440 can project through the housing 452 to provide access to ports 442 (FIG. 4F) that can be used to couple the generator assembly to other devices (e.g., a neuromodulation device, USB, return electrode, monitor, remote control device, etc.). The generator assembly 402 can also include a power plug and/or additional connection ports positioned elsewhere on the housing 452.

The generator assembly 402 can be permanently or semi-permanently mounted to a support structure, such as a patient table, in an equipment rack, and/or on another suitable support structure. FIG. 4G, for example, is a perspective view of the generator assembly 402 mounted to an underside of a patient table 401 in accordance with an embodiment of the present technology. In the illustrated embodiment, the generator assembly 402 is operably coupled to a cable interface module 456 configured to connect the generator assembly 402 with a neuromodulation device (not shown), the remote control device 430, a display 405 spaced apart from the generator assembly 402, and/or other peripheral devices. The cable interface module 456 can be positioned in an easily accessible location, such as on a pole 407 proximate the patient table 401 and may include a convenient place to store the remote control device 430. In other embodiments, peripheral devices can be coupled directly to the generator assembly 402 via the cable interface 440 (FIG. 4F).

The remote display 405 can serve as the display for the system 400 and can communicate the operating conditions of the generator assembly 402 to the user. In the embodiment illustrated in FIG. 4G, for example, the display 405 includes a picture-in-picture (PIP) inset 458 on which the operating conditions are displayed, leaving the remainder of the display 405 free to show other information (e.g., fluoroscopic image guidance displays). In other embodiments, the generator assembly 402 can be coupled to a dedicated display and/or the remote control device 430 that can also display operational conditions to supplement the information shown on the display 405.

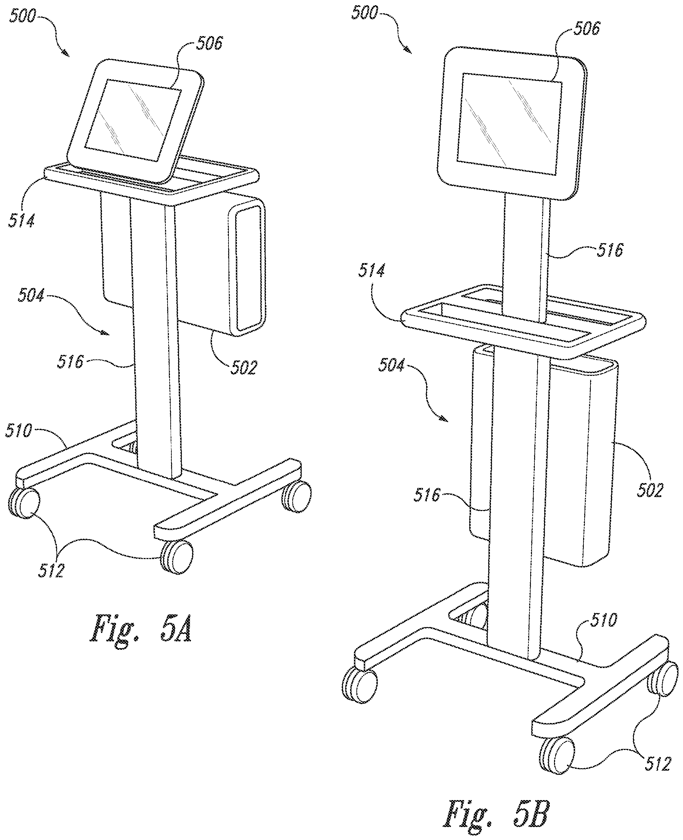

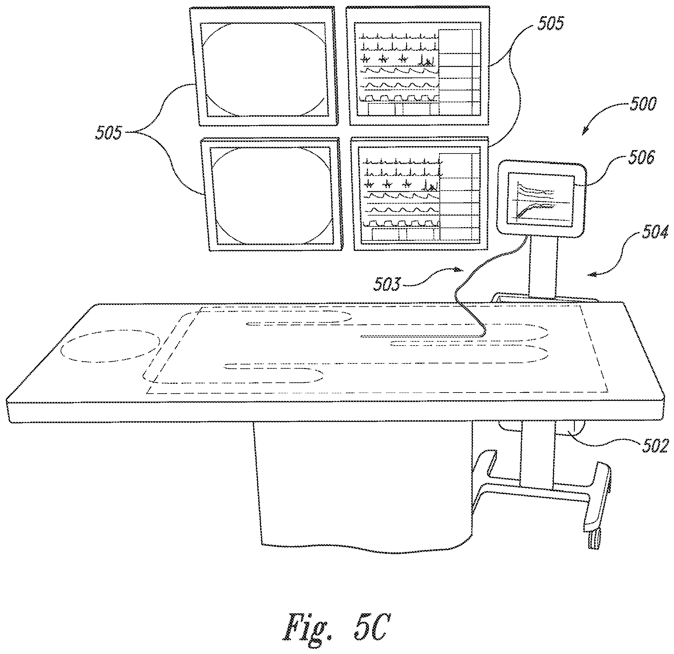

FIGS. 5A and 5B are isometric views of a generator system 500 ("system 500") not showing all features of the claimed invention, and FIG. 5C is a perspective view of the system 500 of FIGS. 5A and 5B in a clinical setting. The system 500 includes a number of features generally similar to the features of the systems 300 and 400 described above with reference to FIGS. 3A-4G. For example, the system 500 includes a generator assembly 502 and a display 506 mounted on a stand assembly 504. Similar to the system 400 of FIGS. 4A-4G, the generator assembly 502 and the display 506 are spaced apart from one another, and the generator assembly 502 can be detached from the stand assembly 504 for use as a standalone device.

The stand assembly 504 can include a maneuverable base portion 510 and a support member 516 extending therefrom to which the generator assembly 502 is attached. The generator assembly 502 can be carried by the support member 516 in a lateral orientation (e.g., as shown in FIG. 5A), a longitudinal orientation (e.g., as shown in FIG. 5B), and/or other suitable orientations. The support member 516 can also carry the display 506 and can be configured to lower the display 506 (e.g., as shown in FIG. 5A), raise the display 506 (e.g., as shown in FIG. 5B), and/or align the display 506 with other monitors 505 (e.g., as shown in FIG. 5C). The display 506 may be lowered, for example, to accommodate operators in a seated position and/or provide for compact storage, and may be raised to, for example, accommodate operators in a standing position.

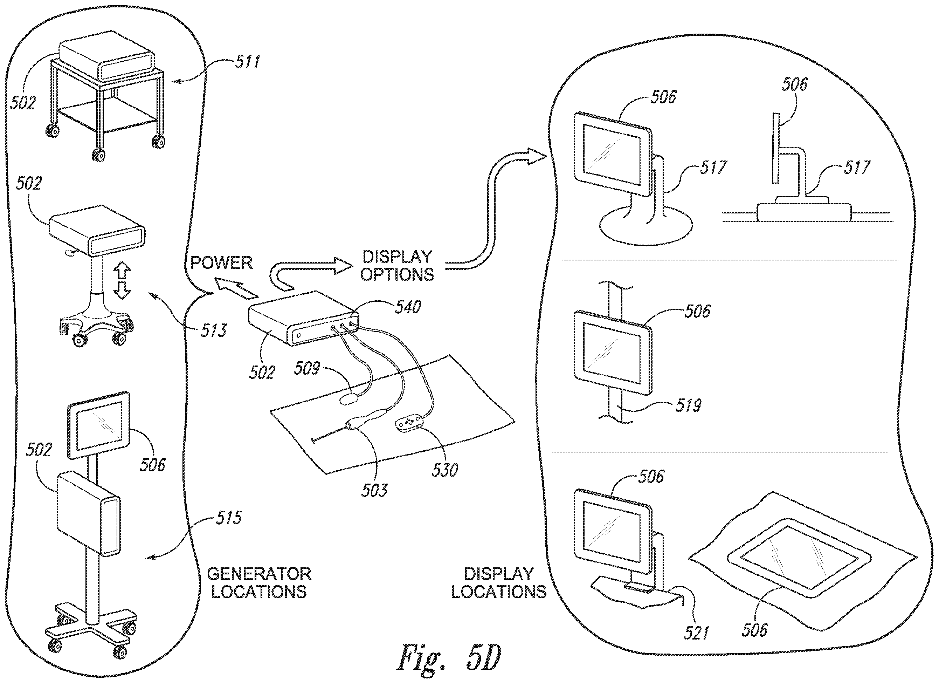

FIG. 5D is a conceptual illustration of various modular configurations of the system 500 of FIGS. 5A and 5B. As shown in FIG. 5D, the generator assembly 502 can be positioned on a cart or table 511, a dedicated stand 513 that can be adjusted per operator preference, or the generator assembly 502 can be mounted to a pole 515 (e.g., an IV pole) with the display 506 optionally mounted overhead. Similarly, the display 506 can also accommodate various configurations. For example, the display 506 can be mounted on or otherwise attached to a stand 517, a pole 519, a bed rail 521, and/or other suitable structures. In any of these configurations, the generator assembly 502 can be operably coupled to a neuromodulation device 503, a remote control device 530, a neutral or dispersive electrode 509, and/or other suitable peripheral devices via a cable interface 540 and provide energy (e.g., RF energy) for neuromodulation procedures.

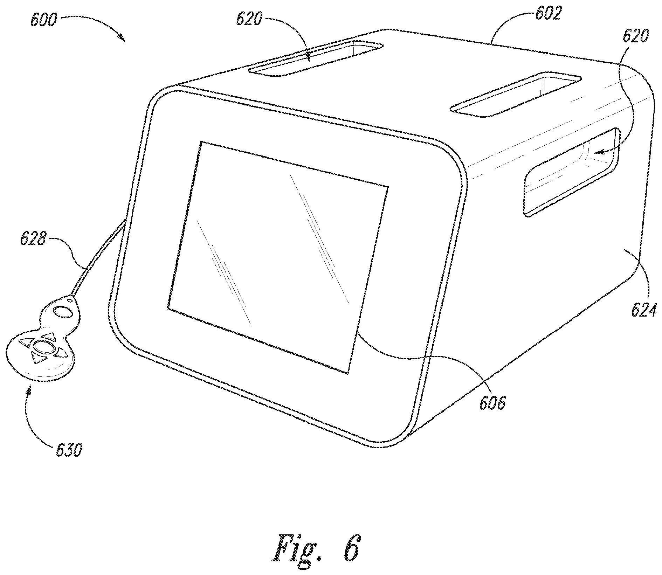

FIG. 6 is an isometric view of a generator system 600 ("system 600") that is not claimed. The system 600 can include features generally similar to the features of the systems 300, 400 and 500 described above with reference to FIGS. 3A-5D. However, the system 600 is a standalone console that integrates a generator assembly 602 and a display 606 into a single housing 624. As shown in FIG. 6, the system 600 can include handle portions 620 to facilitate transport and a remote control device 630 to initiate and control energy delivered by the generator assembly 602. The all-in-one system 600 may not require a stand assembly, and therefore may provide for substantially compact storage on a shelf or in a cabinet.

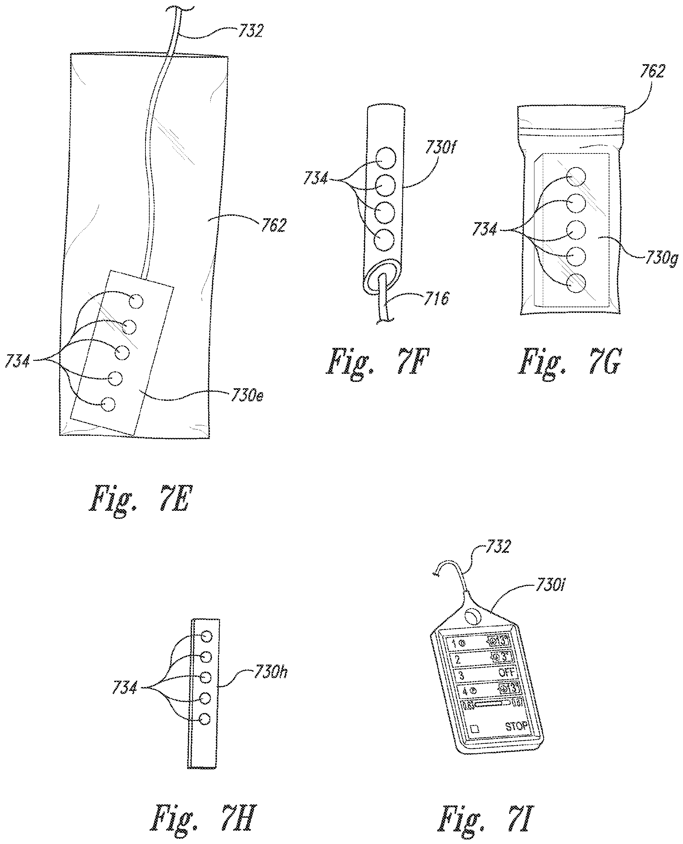

FIGS. 7A-7I illustrates a plurality of remote control devices (identified individually as remote control devices 730a-i, respectively, and referred to collectively as remote control devices 730) for use with generator systems configured in accordance with embodiments of the present technology, such as the generator systems 300-600 described above. The remote control devices 730 can be disposable, non-disposable, hardwired to a generator assembly (e.g., the generator assemblies 302, 402, 502 and 602 described above) via a cable 732 (FIGS. 7A-7D, 7H, and 7I), and/or wirelessly coupled to a generator assembly. For example, the remote control device 730a of FIG. 7A is a foot pedal that can be pressed or otherwise manipulated to activate energy delivery. The first remote control device 730a can be pneumatically or electrically coupled to the generator assembly via the cable 732 and, in various embodiments, may be positioned on a designated portion of a stand assembly (e.g., the stand assembly 304 of FIGS. 3A and 3B) such that the foot pedal moves with the generator system.

As shown in FIGS. 7B-7I, in other embodiments, the remote control devices 730 can be handheld devices that include a plurality of buttons 734, 736 for activating the generator system and controlling energy delivery. Referring to FIGS. 7B-7D, for example, the remote control devices 730b, 730c, 730d, respectively, can include activation and/or selector buttons 734 oriented in a circular pattern (FIGS. 7B and 7C), a linear pattern (FIG. 7D), and/or another easily identifiable button configuration that allows the operator to selectively control energy delivery via a plurality of energy delivery channels without having to look down at the remote control devices 730b, 730c, 730d. In various embodiments, the buttons 734 are backlit to indicate when the corresponding energy delivery channel has been selected, and in some embodiments, each button 734 may be selectively backlit with various different colors that represent an operating condition of the energy delivery channel. For example, a selected button 734 can be backlit a first color (e.g., green) during energy delivery, and can change to a different color (e.g., yellow, red) when an operating parameter of the energy delivery channel falls outside a predetermined operating condition range. As shown in FIG. 7E, the remote control device 730e can be a disposable hardwired controller that can be enclosed in a bag 762 for use in a sterile field. As shown in FIG. 7F, in certain embodiments the remote control device 730f can be mounted on or otherwise integrated with a catheter 716 of a neuromodulation device (e.g., the elongated shaft 116 shown in FIG. 1), and can include finger switches, buttons 734, and/or other actuators to control energy delivery. When integrated with the neuromodulation device, the remote control device 730f can be sterilized along with the neuromodulation device for use in the sterile field, and provide a convenient control means for the operator who may need to manipulate both the remote control device 730e and the neuromodulation device simultaneously or approximately simultaneously.

As shown in FIGS. 7G and 7H, in some embodiments the remote control devices 730g, 730h can be wireless devices communicatively coupled to the generator system, and therefore increase the flexibility of the system. The remote control device 730g of FIG. 7G, for example, is a durable device that can be concealed in a sterile bag 762 for use in a sterile field, whereas the remote control device 730h of FIG. 7H is disposable and can be discarded after use.

As shown in FIG. 7I, in other embodiment the remote control device 730i can include a touchscreen (e.g., similar to the features of the remote control device 430 described above with reference to FIGS. 4A-4G) that can be used to activate the generator system and control energy delivery. The touchscreen remote control device 730i can also provide displays of the operating characteristics of the generator system and/or the neuromodulation device operatively coupled thereto.

Although only some of the remote control devices 730 shown in FIGS. 7A-7I are enclosed in a sterile bag 762, it will be understood that any of the other remote control configurations can also be enclosed in such a bag for use in the sterile field. Additionally, a person skilled in the art will understand that the remote control devices 730 can have various other configurations for controlling energy delivery. For example, the features of one of the remote control devices 730 shown in FIGS. 7A-7I can be combined with the features of another remote control device 730, and/or that some of the features of the remote control devices 730 can be omitted. In other embodiments, the features of the remote control devices 730 described above can be integrated in a handle of a neuromodulation device (e.g., the handle assembly 134 shown in FIG. 1).

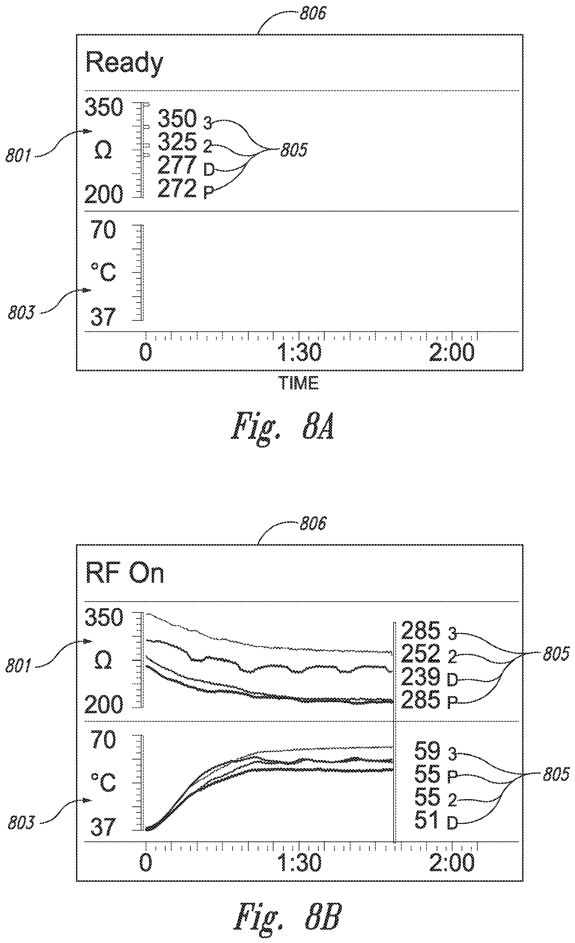

FIGS. 8A-11B are a series of screen shots illustrating various displays for generator systems configured in accordance with aspects of the present technology. The displays can be viewed on any of the displays 306-606 described above, on separate monitors or screens in a clinical setting, a touchscreen of a remote control device, and/or on other suitable devices. For example, FIGS. 8A-8C illustrate screen shots on a display 806 configured in accordance with an embodiment of the present technology. The display 806 includes (1) an impedance vs. time graph 801 used to plot the impedance of RF channels corresponding to one or more energy delivery elements (e.g., electrodes) on a neuromodulation device, and (2) a temperature vs. time graph used to plot the temperature at each electrode during the treatment procedure. The graphs can be updated in real time to provide the operator with feedback before, during, and after energy delivery.

As shown in FIGS. 8A-8C, the impedance and temperature graph 801 can include a plurality of indicators 805 that distinguish four RF channels (e.g., corresponding to four electrodes) from each another. In the illustrated embodiment, for example, a proximal electrode is identified as "P," a distal electrode is identified as "D," and intermediate electrodes (i.e., electrodes between the proximal and distal electrodes) are identified as "2" and "3." In other embodiments, the display 806 can include other indicators to represent the different RF channels (e.g., different colors, symbols, etc.), and include more or fewer indicators depending upon the number of RF channels provided by the generator assembly. In various embodiments, the generator assembly can use an RFID system (e.g., the RFID module 351 of FIGS. 3F-3I) to identify the type of neuromodulation catheter connected to the generator (e.g., a 1, 2, 3...*n* - electrode catheter), and assign the RF channels and associated identifiers (e.g., P, D, etc.) according to the electrode configuration of the neuromodulation catheter.

Referring to FIG. 8A, during an initial stage of a neuromodulation procedure (i.e., before energy delivery), the display 806 can numerically indicate the impedance of each electrode. An operator can use this information to determine whether proper contact has been made between the electrodes and tissue at the target site. For example, a relatively high and steady impedance measurement is thought to correspond to stable tissue contact, and dips in impedance are thought to represent unstable contact with the tissue (e.g., because in some instances the electrode is exposed to more blood flowing through the vessel and blood is expected to have a higher conductivity than tissue). As further shown in FIG. 8A, the display 806 can also include a status indicator message (e.g., "Ready" message) that communicates information to the operator regarding the status of the system. For example, the display shows a "Ready" message to indicate that the generator system is ready to transmit RF energy to the electrodes and/or that the electrodes are in sufficient contact with tissue at the target site (e.g., based on the measured impedances of each electrode).

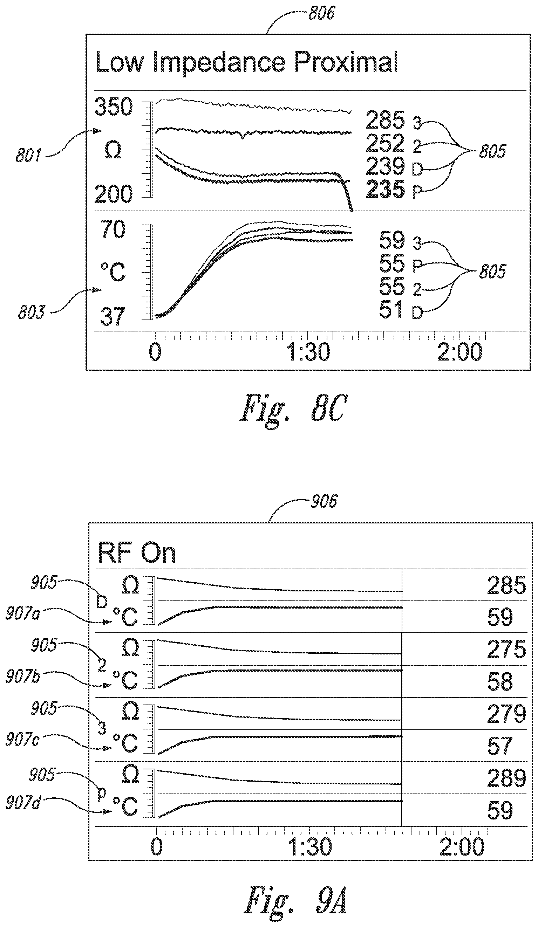

FIG. 8B illustrates the display 806 during an energy application stage of the treatment procedure (e.g., as indicated by the "RF On" message on the display 806). The impedance and temperature of each electrode can be plotted in real time as a separate curve on the impedance and temperature graphs 801 and 803. This allows the operator to track the operating conditions of all displayed electrodes during energy delivery. As shown in FIG. 8C, when one of the electrodes goes outside of a predetermined impedance or temperature range, the display 806 can notify the user of the change in operating condition by displaying a warning message (e.g., "Low Impedance Proximal") and/or highlighting the change in the associated curve on the appropriate graph 801, 803. In certain embodiments, the display 806 can be configured to display a variety of messages or codes (e.g., safety codes, warning messages, TOOR codes, changes in status, etc.) related to operation of the system, and/or provide instructions that provide the appropriate steps to take in response to a specific code. For example, the display 806 can provide visual or audible signals that indicate when an electrode and/or another portion of the system is nearing or outside of a predetermined threshold (e.g., a predetermined temperature range, a predetermined RF level, a predetermined power level, etc.), and provide instructions to correct the deviation outside of the predetermined parameter, such as instructions to adjust RF levels, re-position an electrode, change a catheter, terminate treatment, etc. In other embodiments, the display 806 may visually indicate different information and/or have a different arrangement.

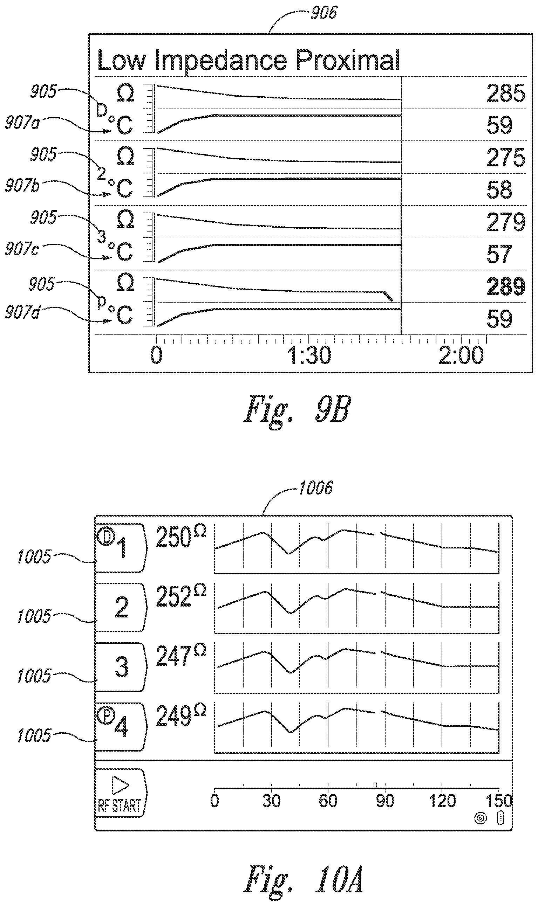

FIGS. 9A and 9B are screen shots illustrating a generator display 906 configured in accordance with other aspects of the present technology. The display 906 includes features generally similar to the features of the display 806 shown in FIGS. 8A-8C. For example, the display 906 includes impedance and temperature plots vs. time for each electrode corresponding to an RF channel, and is configured to indicate when one of the electrodes goes outside a predefined operating range (e.g., as shown in FIG. 9B). However, rather than grouping the impedance plots of all the electrodes together on a single impedance vs. time graph and the temperature plots of all the electrodes together on a single temperature vs. time graph, the display 906 of FIGS. 9A and 9B selectively groups the impedance and temperature vs. time graphs for each electrode. For example, referring to FIG. 9A, each electrode (e.g., D, 2, 3, P) can be associated with an indicator 905 and have an individual graph (identified individually as first through fourth graphs 907a-907d, respectively) associated with it that plots the impedance and temperature of the corresponding electrode vs. time. The current temperature and/or impedance of each electrode can also be numerically displayed. In various embodiments, the display 906 can be configured to allow the operator to navigate between the RF channel-specific plots shown in FIGS. 9A and 9B and the parameter-specific plots shown in FIGS. 8A-8C. In further embodiments, the displays 806, 906 can include different and/or additional graphs associated with other operating parameters (e.g., power).

FIGS. 10A-10D are a series of screen shots illustrating a display 1006 configured in accordance with further aspects of the present technology. The display 1006 can include RF channel indicators 1005 (e.g., 1, 2, 3, 4...n) corresponding to one or more electrodes coupled thereto. As shown in FIG. 10A, before RF energy application, the display 1006 can numerically display the impedance of each electrode and provide a graphical display of the impedance vs. time associated with each electrode (e.g., over a two minute period) to show patterns and thereby indicate whether sufficient contact has been made and maintained at the treatment site. In various embodiments, the display 1006 can be a touchscreen and can accordingly include command buttons that control energy delivery to the electrodes, such as an "RF START" button (FIG. 10A) that can be used to initiate RF energy delivery.

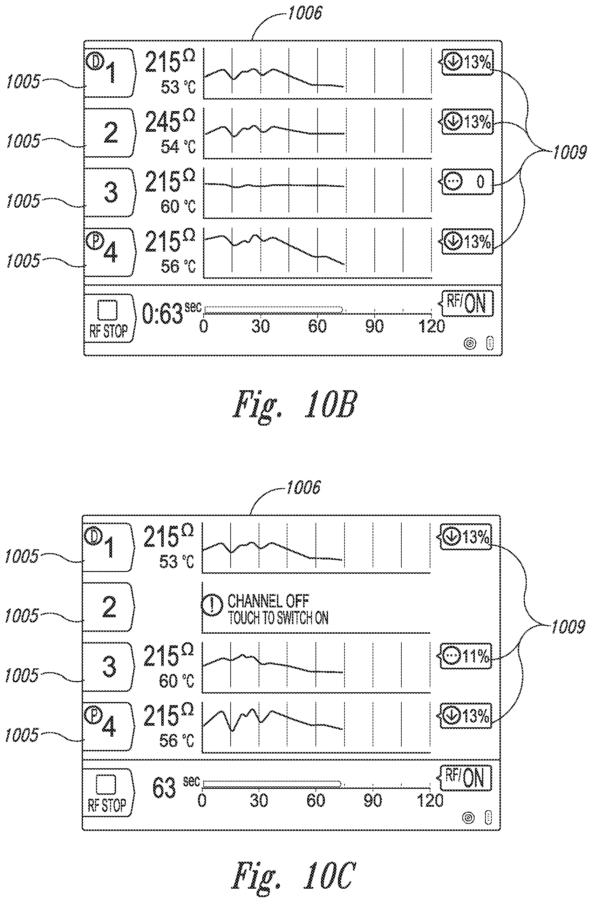

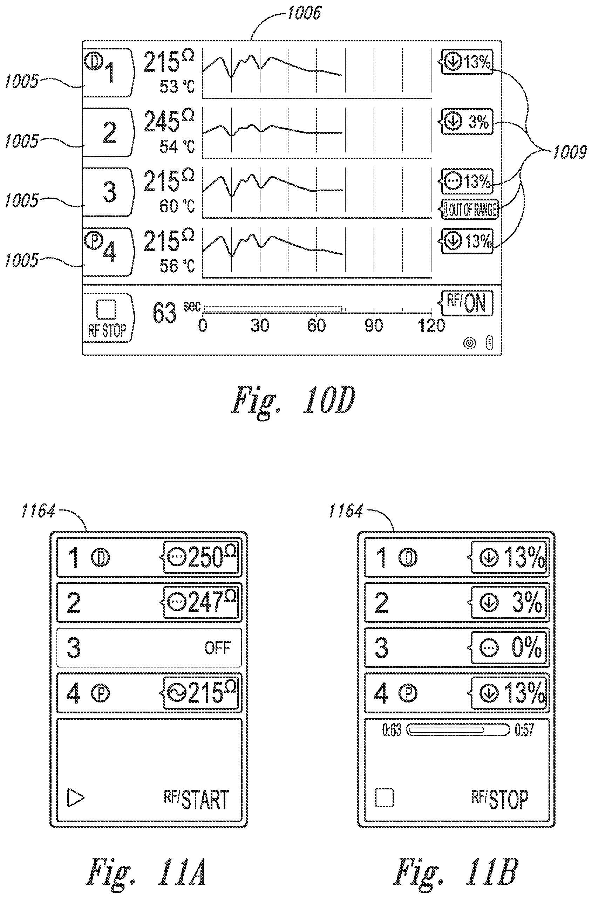

During RF energy delivery, the display 1006 can include a timer that indicates the elapsed time (FIG. 10B) and/or treatment time remaining and an "RF STOP" button that can be used to terminate energy delivery. The display 1006 can also include real time numerical displays of impedance and temperature measurements corresponding to each electrode for quick reference checks, and corresponding graphs that plot changes in impedance in real time. As further shown in FIG. 10B, the display 1006 can also include additional indicators 1009 (e.g., down 13%, down 3%, etc.) that identify patterns or changes in the operating conditions for the operator. The display 1006 can further provide a display or other signal regarding the state of each RF channel, such as when a channel is not turned on (FIG. 10C), and/or indicate when an RF channel is outside the predetermined operating conditions (FIG. 10D). For example, the display 1006 can provide a warning that indicates when an RF channel is outside a predetermined temperature range before the channel is automatically switched off. In other embodiments, the display 1006 can include other and/or additional features, such as plots (e.g., temperature vs. time plots) associated with each RF channel.

FIGS. 11A and 11B are screen shots of a touchscreen 1164 on a remote control device (e.g., the remote control device 430 of FIGS. 4A-4G) configured in accordance with aspects of the present technology. The touchscreen 1164 can be communicatively coupled or synchronized to a larger display (e.g., the display 1006 of FIGS. 10A-10D) and provide information (e.g., operating conditions of electrodes) in an abbreviated format. As shown in FIG. 11A, before energy delivery the touchscreen 1164 can display the activation status and current impedance of each RF channel and provide visual signals as to whether or not the RF channel is stable. For example, if the generator system to which the touchscreen 1164 is coupled detects that the RF channel has not made sufficient contact at the target site, the touchscreen 1164 can provide a visual indicator to that effect (e.g., the symbol shown adjacent the numerical impedance value on RF channel 4). Once the system is sufficiently stable, the operator can begin RF energy delivery by pressing an "RF/START" button on the touchscreen 1164. During energy delivery, the touchscreen 1164 can display the procedure time (FIG. 11B) and indicate the changes in impedance of each RF channel. At any point during the procedure, the operator can press the "RF/STOP" button to terminate energy delivery.

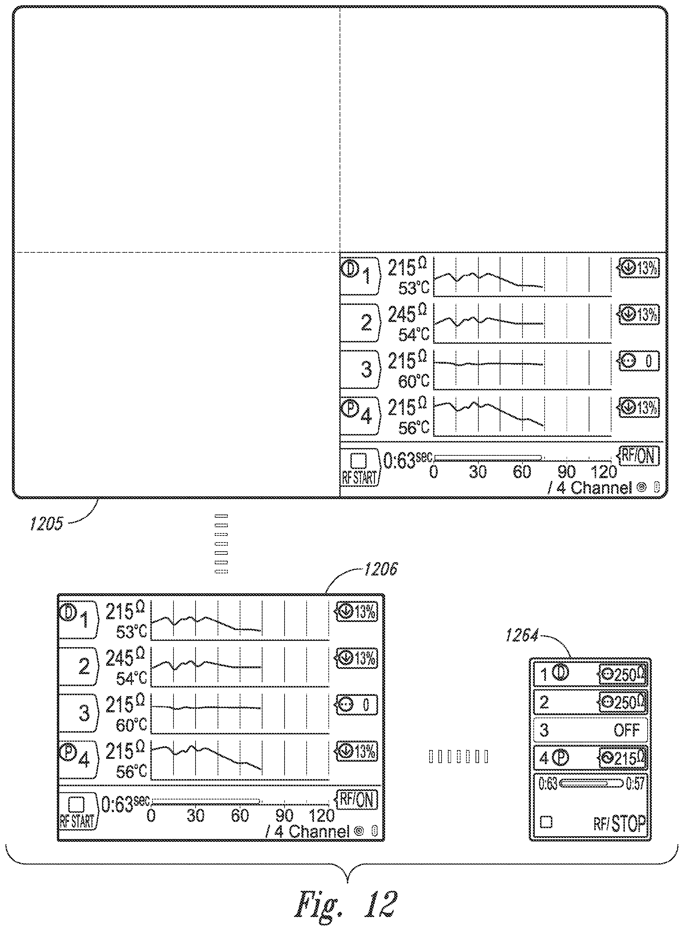

In various embodiments, various displays of a neuromodulation system can be integrated to provide various viewing options. For example, FIG. 12 illustrates the integration of a remote screen 1205, a display 1206 of a generator system, and a touchscreen 1264 of a remote control device in accordance with an embodiment of the present technology. The display 1206 can be replicated as a picture-in-picture inset on a portion of the larger screen 1205 (e.g., positioned in a clinical setting or in a remote lab) and the touchscreen 1264 can display condensed information from the display 1206. As such, the display system can provide a plurality of viewing options for the operator and other users to facilitate monitoring neuromodulation procedures.

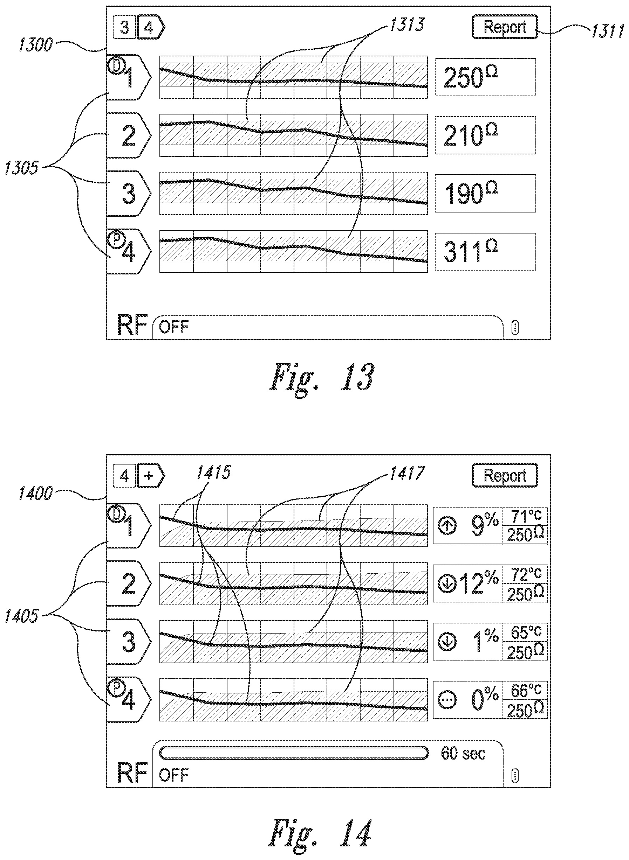

FIG. 13 is a screen shot illustrating a display 1300 configured in accordance with further aspects of the present technology. The display 1300, for example, may be utilized for monitoring impedance stability to determine electrode contact with a vessel wall before RF energy delivery. The display 1300 can include RF channel indicators 1305 (e.g., 1, 2, 3, *4...n)* corresponding to one or more electrodes coupled thereto. In various embodiments, the display 1300 can be a touchscreen and may include command buttons, such as buttons to turn channels ON/OFF, and/or a "REPORT" button 1311 that can be used to display various treatment reports and associated data. The display 1300 can also be configured to show a number of past RF ablations performed in the current treatment and/or using the current catheter. Before RF energy delivery, the display 1300 is configured to provide a numerical impedance display for each RF channel and a graphical display of the impedance vs. time for each RF channel over a continuous, selected period (e.g., 30 seconds). The display 1300, for example, can include a dynamic rolling display that is updated at periodic intervals to provide an operator with historical tracking of impedance measurements. The display 1300 can also include real time numerical displays of impedance measurements corresponding to each RF channel for quick reference checks. The dynamic rolling display is configured to show trends/patterns in the impedance for each RF channel to assist the operator in assessing that stable contact has been made and maintained by the individual electrodes at the treatment site. Further, such indications can alert the operator of a contrast injection that is affecting the measurement(s) or that one or more of the electrodes may be unstable.

The display 1300 can provide an indication of catheter position and/or stability to an operator by displaying a range of impedance values as a background color bar 1313 to the current impedance value. The range, for example, could be from various statistical indicators such as standard deviation, maximum and minimum, or any combination of statistical indicators. In some embodiments, the display 1300 can also include other numerical displays such as current temperature, standard deviation of a selected period of impedance, mean value of a selected period of impedance, and the like. Along with numerical indicators, the display 1300 may also include additional graphical indicators to instruct the operator to proceed with treatment, not proceed with treatment, adjust the position of one or more of the electrodes, etc. The display 1300 may also show conditions such as catheter in motion, catheter deployed, catheter disconnected, and/or other catheter stability measures.

The display 1300 can further provide a clear signal regarding the state of each RF channel, such as when a channel is not turned on (e.g., based on a color of the channel number indicator) and/or indications when an RF channel is outside a predetermined operating condition (by changing the color of the channel number indicator). In other embodiments, the display 1300 can include other and/or additional features, such as plots (e.g., temperature vs. time plots) associated with each RF channel.

FIG. 14 is a screen shot illustrating a display 1400 configured in accordance with further aspects of the present technology. The display 1400, for example, may be utilized for viewing impedance and temperature together (e.g., overlaid) over a continuous, selected period (e.g., 60 seconds) during RF energy delivery. The display 1400 can include a number of features generally similar to the other displays described herein. For example, the display 1400 can include RF channel indicators 1405 (e.g., 1, 2, 3, 4...n) corresponding to one or more electrodes coupled thereto. Before RF energy delivery, the display 1400 is configured to provide a dynamic rolling display of impedance measurements for each channel overlaid with temperature measurements for each channel. In some embodiments, impedance measurements 1415 may be emphasized or highlighted over the corresponding temperature measurements 1417. For example, the impedance measurement 1415 can be emphasized based, at least in part, on its arrangement relative to the temperature bar 1417 and the color used to display the impedance measurement (e.g., the temperature measurement is shown behind the impedance line and in a different color). In this example, the height of the temperature bar 1417 for each RF channel can be used to provide a visual reference of the changes/trends in temperature at the treatment site.

For each RF channel during the selected period, the display 1400 can also include (a) real time numerical displays of a percentage (%) change in impedance measurement, (b) real time numerical displays of impedance measurements for quick reference checks of total impedance for particular channels, and (c) real time numerical displays of temperature for each RF channel. In other embodiments, the display 1400 may display a different set of data and/or have a different arrangement. The overlaid arrangement of data shown in the display 1400 is expected to provide the operator with a variety of useful information to quickly and accurately assess the treatment conditions, and to help the operator ensure that stable contact has been made and maintained by the individual electrodes at the treatment site before RF delivery. In other embodiments, however, the display 1400 may have a different arrangement and/or may include other features. Further, the display 1400 can be configured to display information before and/or after RF energy delivery.

As a person of skill in the art will readily appreciate, any of the displays 305, 306, 405, 406, 464, 505, 506, 606, 806, 906, 1006, 1106, 1205, 1206, 1264, 1300, and 1400 described above with reference to FIGS. 3A-14 can be configured to display (e.g., numerically and/or graphically) other characteristics/parameters detected or otherwise determined by the associated systems 300, 300J, 400, 500, and 600 and/or extensions thereof. For example, the displays can be can be configured to provide blood flow measurements, blood pressure measurements, pressure measurements associated with each energy delivery element, the contact force sensed by each energy delivery element as it presses against adjacent tissue, and/or various other characteristics associated with and pertinent to neuromodulation therapy. These characteristics/parameters can be displayed numerically and/or graphically (e.g., as a dynamic rolling display) in substantially real time before, during, and/or after a therapeutic procedure. In addition, in certain embodiments the display can be configured to indicate when one of the detected characteristic/parameter is close to or outside of a predetermined threshold by emitting a warning sound, providing another audible warning message, and/or displaying a warning message, code, or icon.

### IV. Related Anatomy and Physiology

As noted previously, the sympathetic nervous system (SNS) is a branch of the autonomic nervous system along with the enteric nervous system and parasympathetic nervous system. It is always active at a basal level (called sympathetic tone) and becomes more active during times of stress. Like other parts of the nervous system, the sympathetic nervous system operates through a series of interconnected neurons. Sympathetic neurons are frequently considered part of the peripheral nervous system (PNS), although many lie within the central nervous system (CNS). Sympathetic neurons of the spinal cord (which is part of the CNS) communicate with peripheral sympathetic neurons via a series of sympathetic ganglia. Within the ganglia, spinal cord sympathetic neurons join peripheral sympathetic neurons through synapses. Spinal cord sympathetic neurons are therefore called presynaptic (or preganglionic) neurons, while peripheral sympathetic neurons are called postsynaptic (or postganglionic) neurons.

At synapses within the sympathetic ganglia, preganglionic sympathetic neurons release acetylcholine, a chemical messenger that binds and activates nicotinic acetylcholine receptors on postganglionic neurons. In response to this stimulus, postganglionic neurons principally release noradrenaline (norepinephrine). Prolonged activation may elicit the release of adrenaline from the adrenal medulla.

Once released, norepinephrine and epinephrine bind adrenergic receptors on peripheral tissues. Binding to adrenergic receptors causes a neuronal and hormonal response. The physiologic manifestations include pupil dilation, increased heart rate, occasional vomiting, and increased blood pressure. Increased sweating is also seen due to binding of cholinergic receptors of the sweat glands.

The sympathetic nervous system is responsible for up- and down-regulating many homeostatic mechanisms in living organisms. Fibers from the SNS innervate tissues in almost every organ system, providing at least some regulatory function to physiological features as diverse as pupil diameter, gut motility, and urinary output. This response is also known as *sympatho-adrenal response* of the body, as the preganglionic sympathetic fibers that end in the adrenal medulla (but also all other sympathetic fibers) secrete acetylcholine, which activates the secretion of adrenaline (epinephrine) and to a lesser extent noradrenaline (norepinephrine). Therefore, this response that acts primarily on the cardiovascular system is mediated directly via impulses transmitted through the sympathetic nervous system and indirectly via catecholamines secreted from the adrenal medulla.

Science typically looks at the SNS as an automatic regulation system, that is, one that operates without the intervention of conscious thought. Some evolutionary theorists suggest that the sympathetic nervous system operated in early organisms to maintain survival as the sympathetic nervous system is responsible for priming the body for action. One example of this priming is in the moments before waking, in which sympathetic outflow spontaneously increases in preparation for action.

### 1. The Sympathetic Chain

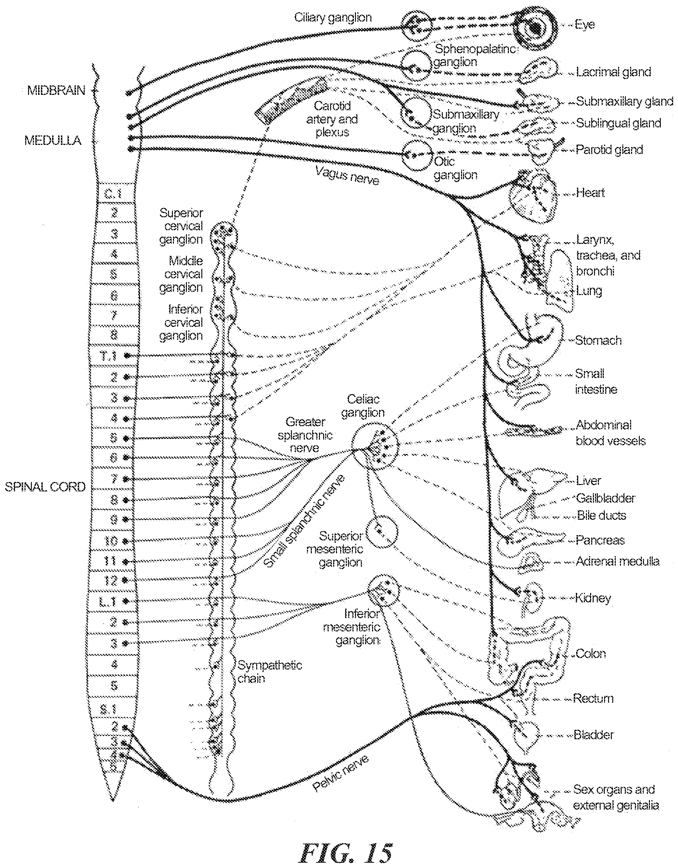

As shown in FIG. 15, the SNS provides a network of nerves that allows the brain to communicate with the body. Sympathetic nerves originate inside the vertebral column, toward the middle of the spinal cord in the intermediolateral cell column (or lateral horn), beginning at the first thoracic segment of the spinal cord and are thought to extend to the second or third lumbar segments. Because its cells begin in the thoracic and lumbar regions of the spinal cord, the SNS is said to have a thoracolumbar outflow. Axons of these nerves leave the spinal cord through the anterior rootlet/root. They pass near the spinal (sensory) ganglion, where they enter the anterior rami of the spinal nerves. However, unlike somatic innervation, they quickly separate out through white rami connectors which connect to either the paravertebral (which lie near the vertebral column) or prevertebral (which lie near the aortic bifurcation) ganglia extending alongside the spinal column.

In order to reach the target organs and glands, the axons should travel long distances in the body, and, to accomplish this, many axons relay their message to a second cell through synaptic transmission. The ends of the axons link across a space, the synapse, to the dendrites of the second cell. The first cell (the presynaptic cell) sends a neurotransmitter across the synaptic cleft where it activates the second cell (the postsynaptic cell). The message is then carried to the final destination.

In the SNS and other components of the peripheral nervous system, these synapses are made at sites called ganglia, discussed above. The cell that sends its fiber is called a preganglionic cell, while the cell whose fiber leaves the ganglion is called a postganglionic cell. As mentioned previously, the preganglionic cells of the SNS are located between the first thoracic (T1) segment and third lumbar (L3) segments of the spinal cord. Postganglionic cells have their cell bodies in the ganglia and send their axons to target organs or glands.

The ganglia include not just the sympathetic trunks but also the cervical ganglia (superior, middle and inferior), which sends sympathetic nerve fibers to the head and thorax organs, and the celiac and mesenteric ganglia (which send sympathetic fibers to the gut).

### 2. Innervation of the Kidneys

As FIG. 16 shows, the kidney is innervated by the renal plexus (RP), which is intimately associated with the renal artery. The renal plexus (RP) is an autonomic plexus that surrounds the renal artery and is embedded within the adventitia of the renal artery. The renal plexus (RP) extends along the renal artery until it arrives at the substance of the kidney. Fibers contributing to the renal plexus (RP) arise from the celiac ganglion, the superior mesenteric ganglion, the aorticorenal ganglion and the aortic plexus. The renal plexus (RP), also referred to as the renal nerve, is predominantly comprised of sympathetic components. There is no (or at least very minimal) parasympathetic innervation of the kidney.

Preganglionic neuronal cell bodies are located in the intermediolateral cell column of the spinal cord. Preganglionic axons pass through the paravertebral ganglia (they do not synapse) to become the lesser splanchnic nerve, the least splanchnic nerve, first lumbar splanchnic nerve, second lumbar splanchnic nerve, and travel to the celiac ganglion, the superior mesenteric ganglion, and the aorticorenal ganglion. Postganglionic neuronal cell bodies exit the celiac ganglion, the superior mesenteric ganglion, and the aorticorenal ganglion to the renal plexus (RP) and are distributed to the renal vasculature.

### 3. Renal Sympathetic Neural Activity

Messages travel through the SNS in a bidirectional flow. Efferent messages may trigger changes in different parts of the body simultaneously. For example, the sympathetic nervous system may accelerate heart rate; widen bronchial passages; decrease motility (movement) of the large intestine; constrict blood vessels; increase peristalsis in the esophagus; cause pupil dilation, piloerection (goose bumps) and perspiration (sweating); and raise blood pressure. Afferent messages carry signals from various organs and sensory receptors in the body to other organs and, particularly, the brain.

Hypertension, heart failure and chronic kidney disease are a few of many disease states that result from chronic activation of the SNS, especially the renal sympathetic nervous system. Chronic activation of the SNS is a maladaptive response that drives the progression of these disease states. Pharmaceutical management of the renin-angiotensin-aldosterone system (RAAS) has been a longstanding, but somewhat ineffective, approach for reducing over-activity of the SNS.

As mentioned above, the renal sympathetic nervous system has been identified as a major contributor to the complex pathophysiology of hypertension, states of volume overload (such as heart failure), and progressive renal disease, both experimentally and in humans. Studies employing radiotracer dilution methodology to measure overflow of norepinephrine from the kidneys to plasma revealed increased renal norepinephrine (NE) spillover rates in patients with essential hypertension, particularly so in young hypertensive subjects, which in concert with increased NE spillover from the heart, is consistent with the hemodynamic profile typically seen in early hypertension and characterized by an increased heart rate, cardiac output, and renovascular resistance. It is now known that essential hypertension is commonly neurogenic, often accompanied by pronounced sympathetic nervous system overactivity.

Activation of cardiorenal sympathetic nerve activity is even more pronounced in heart failure, as demonstrated by an exaggerated increase of NE overflow from the heart and the kidneys to plasma in this patient group. In line with this notion is the recent demonstration of a strong negative predictive value of renal sympathetic activation on all-cause mortality and heart transplantation in patients with congestive heart failure, which is independent of overall sympathetic activity, glomerular filtration rate, and left ventricular ejection fraction. These findings support the notion that treatment regimens that are designed to reduce renal sympathetic stimulation have the potential to improve survival in patients with heart failure.

Both chronic and end stage renal disease are characterized by heightened sympathetic nervous activation. In patients with end stage renal disease, plasma levels of norepinephrine above the median have been demonstrated to be predictive for both all-cause death and death from cardiovascular disease. This is also true for patients suffering from diabetic or contrast nephropathy. There is compelling evidence suggesting that sensory afferent signals originating from the diseased kidneys are major contributors to initiating and sustaining elevated central sympathetic outflow in this patient group; this facilitates the occurrence of the well known adverse consequences of chronic sympathetic over activity, such as hypertension, left ventricular hypertrophy, ventricular arrhythmias, sudden cardiac death, insulin resistance, diabetes, and metabolic syndrome.

### (i) Renal Sympathetic Efferent Activity

Sympathetic nerves to the kidneys terminate in the blood vessels, the juxtaglomerular apparatus and the renal tubules. Stimulation of the renal sympathetic nerves causes increased renin release, increased sodium (Na⁺) reabsorption, and a reduction of renal blood flow. These components of the neural regulation of renal function are considerably stimulated in disease states characterized by heightened sympathetic tone and clearly contribute to the rise in blood pressure in hypertensive patients. The reduction of renal blood flow and glomerular filtration rate as a result of renal sympathetic efferent stimulation is likely a cornerstone of the loss of renal function in cardio-renal syndrome, which is renal dysfunction as a progressive complication of chronic heart failure, with a clinical course that typically fluctuates with the patient's clinical status and treatment. Pharmacologic strategies to thwart the consequences of renal efferent sympathetic stimulation include centrally acting sympatholytic drugs, beta blockers (intended to reduce renin release), angiotensin converting enzyme inhibitors and receptor blockers (intended to block the action of angiotensin II and aldosterone activation consequent to renin release) and diuretics (intended to counter the renal sympathetic mediated sodium and water retention). However, the current pharmacologic strategies have significant limitations including limited efficacy, compliance issues, side effects and others.

### (ii) Renal Sensory Afferent Nerve Activity

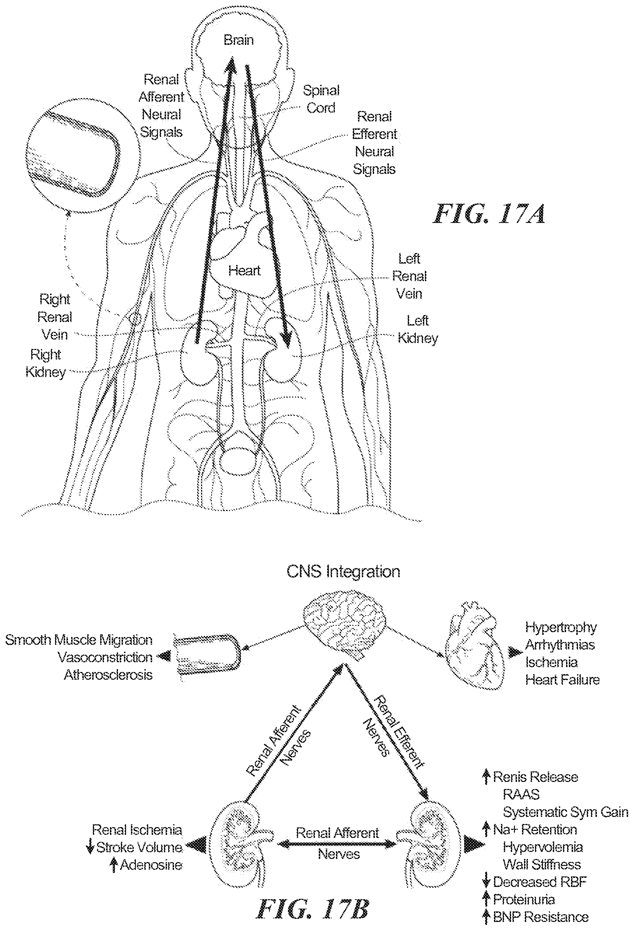

The kidneys communicate with integral structures in the central nervous system via renal sensory afferent nerves. Several forms of "renal injury" may induce activation of sensory afferent signals. For example, renal ischemia, reduction in stroke volume or renal blood flow, or an abundance of adenosine enzyme may trigger activation of afferent neural communication. As shown in FIGS. 17A and 17B, this afferent communication might be from the kidney to the brain or might be from one kidney to the other kidney (via the central nervous system). These afferent signals are centrally integrated and may result in increased sympathetic outflow. This sympathetic drive is directed towards the kidneys, thereby activating the RAAS and inducing increased renin secretion, sodium retention, volume retention and vasoconstriction. Central sympathetic over activity also impacts other organs and bodily structures innervated by sympathetic nerves such as the heart and the peripheral vasculature, resulting in the described adverse effects of sympathetic activation, several aspects of which also contribute to the rise in blood pressure.

The physiology therefore suggests that (i) modulation of tissue with efferent sympathetic nerves will reduce inappropriate renin release, salt retention, and reduction of renal blood flow, and that (ii) modulation of tissue with afferent sensory nerves will reduce the systemic contribution to hypertension and other disease states associated with increased central sympathetic tone through its direct effect on the posterior hypothalamus as well as the contralateral kidney. In addition to the central hypotensive effects of afferent renal denervation, a desirable reduction of central sympathetic outflow to various other sympathetically innervated organs such as the heart and the vasculature is anticipated.

### B. Additional Clinical Benefits of Renal Denervation

As provided above, renal denervation is likely to be valuable in the treatment of several clinical conditions characterized by increased overall and particularly renal sympathetic activity such as hypertension, metabolic syndrome, insulin resistance, diabetes, left ventricular hypertrophy, chronic end stage renal disease, inappropriate fluid retention in heart failure, cardio-renal syndrome, and sudden death. Since the reduction of afferent neural signals contributes to the systemic reduction of sympathetic tone/drive, renal denervation might also be useful in treating other conditions associated with systemic sympathetic hyperactivity. Accordingly, renal denervation may also benefit other organs and bodily structures innervated by sympathetic nerves, including those identified in FIG. 15. For example, as previously discussed, a reduction in central sympathetic drive may reduce the insulin resistance that afflicts people with metabolic syndrome and Type II diabetics. Additionally, patients with osteoporosis are also sympathetically activated and might also benefit from the down regulation of sympathetic drive that accompanies renal denervation.

### C. Achieving Intravascular Access to the Renal Artery

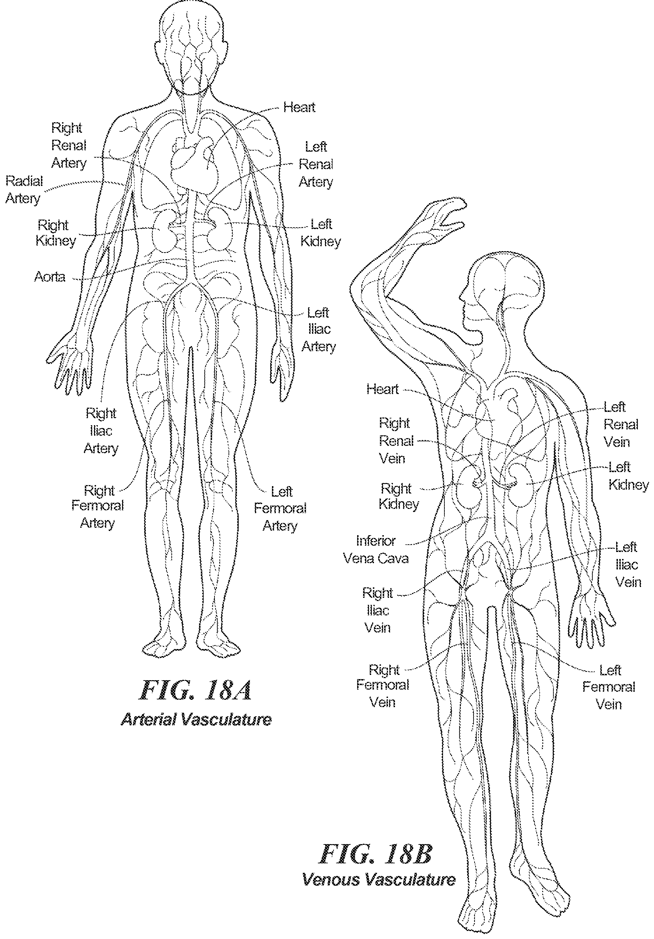

In accordance with the present technology, neuromodulation of a left and/or right renal plexus (RP), which is intimately associated with a left and/or right renal artery, may be achieved through intravascular access. As FIG. 18A shows, blood moved by contractions of the heart is conveyed from the left ventricle of the heart by the aorta. The aorta descends through the thorax and branches into the left and right renal arteries. Below the renal arteries, the aorta bifurcates at the left and right iliac arteries. The left and right iliac arteries descend, respectively, through the left and right legs and join the left and right femoral arteries.

As FIG. 18B shows, the blood collects in veins and returns to the heart, through the femoral veins into the iliac veins and into the inferior vena cava. The inferior vena cava branches into the left and right renal veins. Above the renal veins, the inferior vena cava ascends to convey blood into the right atrium of the heart. From the right atrium, the blood is pumped through the right ventricle into the lungs, where it is oxygenated. From the lungs, the oxygenated blood is conveyed into the left atrium. From the left atrium, the oxygenated blood is conveyed by the left ventricle back to the aorta.

As will be described in greater detail later, the femoral artery may be accessed and cannulated at the base of the femoral triangle just inferior to the midpoint of the inguinal ligament. A catheter may be inserted percutaneously into the femoral artery through this access site, passed through the iliac artery and aorta, and placed into either the left or right renal artery. This comprises an intravascular path that offers minimally invasive access to a respective renal artery and/or other renal blood vessels.

The wrist, upper arm, and shoulder region provide other locations for introduction of catheters into the arterial system. For example, catheterization of either the radial, brachial, or axillary artery may be utilized in select cases. Catheters introduced via these access points may be passed through the subclavian artery on the left side (or via the subclavian and brachiocephalic arteries on the right side), through the aortic arch, down the descending aorta and into the renal arteries using standard angiographic technique.

### D. Properties and Characteristics of the Renal Vasculature

Since neuromodulation of a left and/or right renal plexus (RP) may be achieved in accordance with the present technology through intravascular access, properties and characteristics of the renal vasculature may impose constraints upon and/or inform the design of apparatus, systems, and methods for achieving such renal neuromodulation. Some of these properties and characteristics may vary across the patient population and/or within a specific patient across time, as well as in response to disease states, such as hypertension, chronic kidney disease, vascular disease, end-stage renal disease, insulin resistance, diabetes, metabolic syndrome, etc. These properties and characteristics, as explained herein, may have bearing on the efficacy of the procedure and the specific design of the intravascular device. Properties of interest may include, for example, material/mechanical, spatial, fluid dynamic/hemodynamic and/or thermodynamic properties.

As discussed previously, a catheter may be advanced percutaneously into either the left or right renal artery via a minimally invasive intravascular path. However, minimally invasive renal arterial access may be challenging, for example, because as compared to some other arteries that are routinely accessed using catheters, the renal arteries are often extremely tortuous, may be of relatively small diameter, and/or may be of relatively short length. Furthermore, renal arterial atherosclerosis is common in many patients, particularly those with cardiovascular disease. Renal arterial anatomy also may vary significantly from patient to patient, which further complicates minimally invasive access. Significant inter-patient variation may be seen, for example, in relative tortuosity, diameter, length, and/or atherosclerotic plaque burden, as well as in the take-off angle at which a renal artery branches from the aorta. Apparatus, systems and methods for achieving renal neuromodulation via intravascular access should account for these and other aspects of renal arterial anatomy and its variation across the patient population when minimally invasively accessing a renal artery.

In addition to complicating renal arterial access, specifics of the renal anatomy also complicate establishment of stable contact between neuromodulatory apparatus and a luminal surface or wall of a renal artery. For example, navigation can be impeded by the tight space within a renal artery, as well as tortuosity of the artery. Furthermore, establishing consistent contact is complicated by patient movement, respiration, and/or the cardiac cycle because these factors may cause significant movement of the renal artery relative to the aorta, and the cardiac cycle may transiently distend the renal artery (i.e. cause the wall of the artery to pulse).

Even after accessing a renal artery and facilitating stable contact between neuromodulatory apparatus and a luminal surface of the artery, nerves in and around the adventia of the artery should be safely modulated via the neuromodulatory apparatus. Effectively applying thermal treatment from within a renal artery is non-trivial given the potential clinical complications associated with such treatment. For example, the intima and media of the renal artery are highly vulnerable to thermal injury. As discussed in greater detail below, the intima-media thickness separating the vessel lumen from its adventitia means that target renal nerves may be multiple millimeters distant from the luminal surface of the artery. Sufficient energy should be delivered to or heat removed from the target renal nerves to modulate the target renal nerves without excessively cooling or heating the vessel wall to the extent that the wall is frozen, desiccated, or otherwise potentially affected to an undesirable extent. A potential clinical complication associated with excessive heating is thrombus formation from coagulating blood flowing through the artery. Given that this thrombus may cause a kidney infarct, thereby causing irreversible damage to the kidney, thermal treatment from within the renal artery should be applied carefully. Accordingly, the complex fluid mechanics and thermodynamic conditions present in the renal artery during treatment, particularly those that may impact heat transfer dynamics at the treatment site, may be important in applying energy (e.g., heating thermal energy) and/or removing heat from the tissue (e.g., cooling thermal conditions) from within the renal artery.

The neuromodulatory apparatus should also be configured to allow for adjustable positioning and repositioning of the energy delivery element within the renal artery since location of treatment may also impact clinical efficacy. For example, it may be tempting to apply a full circumferential treatment from within the renal artery given that the renal nerves may be spaced circumferentially around a renal artery. In some situations, a full-circle lesion likely resulting from a continuous circumferential treatment may be potentially related to renal artery stenosis. Therefore, the formation of more complex lesions along a longitudinal dimension of the renal artery and/or repositioning of the neuromodulatory apparatus to multiple treatment locations may be desirable. It should be noted, however, that a benefit of creating a circumferential ablation may outweigh the potential of renal artery stenosis or the risk may be mitigated with certain embodiments or in certain patients and creating a circumferential ablation could be a goal. Additionally, variable positioning and repositioning of the neuromodulatory apparatus may prove to be useful in circumstances where the renal artery is particularly tortuous or where there are proximal branch vessels off the renal artery main vessel, making treatment in certain locations challenging. Manipulation of a device in a renal artery should also consider mechanical injury imposed by the device on the renal artery. Motion of a device in an artery, for example by inserting, manipulating, negotiating bends and so forth, may contribute to dissection, perforation, denuding intima, or disrupting the interior elastic lamina.

Blood flow through a renal artery may be temporarily occluded for a short time with minimal or no complications. However, occlusion for a significant amount of time should be avoided because to prevent injury to the kidney such as ischemia. It could be beneficial to avoid occlusion all together or, if occlusion is beneficial to the embodiment, to limit the duration of occlusion, for example to 2-5 minutes.

Based on the above described challenges of (1) renal artery intervention, (2) consistent and stable placement of the treatment element against the vessel wall, (3) effective application of treatment across the vessel wall, (4) positioning and potentially repositioning the treatment apparatus to allow for multiple treatment locations, and (5) avoiding or limiting duration of blood flow occlusion, various independent and dependent properties of the renal vasculature that may be of interest include, for example, (a) vessel diameter, vessel length, intima-media thickness, coefficient of friction, and tortuosity; (b) distensibility, stiffness and modulus of elasticity of the vessel wall; (c) peak systolic, end-diastolic blood flow velocity, as well as the mean systolic-diastolic peak blood flow velocity, and mean/max volumetric blood flow rate; (d) specific heat capacity of blood and/or of the vessel wall, thermal conductivity of blood and/or of the vessel wall, and/or thermal convectivity of blood flow past a vessel wall treatment site and/or radiative heat transfer; (e) renal artery motion relative to the aorta induced by respiration, patient movement, and/or blood flow pulsatility; and (f) the take-off angle of a renal artery relative to the aorta. These properties will be discussed in greater detail with respect to the renal arteries. However, dependent on the apparatus, systems and methods utilized to achieve renal neuromodulation, such properties of the renal arteries, also may guide and/or constrain design characteristics.

As noted above, an apparatus positioned within a renal artery should conform to the geometry of the artery. Renal artery vessel diameter, D_{RA}, typically is in a range of about 2-10 mm, with most of the patient population having a D_{RA} of about 4 mm to about 8 mm and an average of about 6 mm. Renal artery vessel length, L_{RA}, between its ostium at the aorta/renal artery juncture and its distal branchings, generally is in a range of about 5-70 mm, and a significant portion of the patient population is in a range of about 20-50 mm. Since the target renal plexus is embedded within the adventitia of the renal artery, the composite Intima-Media Thickness, IMT, (i.e., the radial outward distance from the artery's luminal surface to the adventitia containing target neural structures) also is notable and generally is in a range of about 0.5-2.5 mm, with an average of about 1.5 mm. Although a certain depth of treatment is important to reach the target neural fibers, the treatment should not be too deep (e.g., > 5 mm from inner wall of the renal artery) to avoid non-target tissue and anatomical structures such as the renal vein.

An additional property of the renal artery that may be of interest is the degree of renal motion relative to the aorta induced by respiration and/or blood flow pulsatility. A patient's kidney, which is located at the distal end of the renal artery, may move as much as 4" cranially with respiratory excursion. This may impart significant motion to the renal artery connecting the aorta and the kidney, thereby requiring from the neuromodulatory apparatus a unique balance of stiffness and flexibility to **maintain** contact between the energy delivery element and the vessel wall during cycles of respiration. Furthermore, the take-off angle between the renal artery and the aorta may vary significantly between patients, and also may vary dynamically within a patient, e.g., due to kidney motion. The take-off angle generally may be in a range of about 30°-135°.

### VI. Conclusion

The above detailed descriptions of embodiments of the technology are not intended to be exhaustive or to limit the technology to the precise form disclosed above. Although specific embodiments of, and examples for, the technology are described above for illustrative purposes, various equivalent modifications are possible within the scope of the technology, as those skilled in the relevant art will recognize. For example, while steps are presented in a given order, alternative methods may perform steps in a different order. The various embodiments described herein may also be combined to provide further embodiments.

From the foregoing, it will be appreciated that specific embodiments of the technology have been described herein for purposes of illustration, but well-known structures and functions have not been shown or described in detail to avoid unnecessarily obscuring the description of the embodiments of the technology. Where the context permits, singular or plural terms may also include the plural or singular term, respectively.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

## Claims

1. A generator system (300, 400, 500) for neuromodulation therapy, the generator system comprising:
a generator console (326) comprising an energy delivery channel, a display (306), and at least one port (342) configured to operably couple the generator console to a neuromodulation device (112), wherein the generator console is configured to deliver energy via the energy delivery channel to an electrode (124) of the neuromodulation device, and wherein the display is configured to indicate operating conditions of the energy delivery channel during energy delivery; and
a stand assembly (304) configured to carry the generator console, **characterised in that** the stand assembly comprises -
a body portion (308) having a recessed portion (318) configured to receive a portion of the generator console, wherein the recessed portion includes a plurality of spring-loaded locking members (337) protruding away from the recessed portion and movable between a locked arrangement and an unlocked arrangement, and wherein the locking members are configured to releasably attach to the generator console when the locking members are in the locked arrangement;
a base portion (310) supporting the body portion; and
a release mechanism (335) operably coupled to the locking members, wherein the release mechanism is configured to be manipulated to move the locking members between the locked arrangement and the unlocked arrangement to disengage the generator console from the stand assembly,
wherein the locking members are spring-loaded such that the weight of the console on the locking members can automatically latch the locking members to the console as the console is placed on the stand assembly.

2. The generator system (300) of claim 1 wherein the locking members (337) are configured to emit an audible sound as the locking members move to the locked arrangement in connection with the generator console (326), or wherein the recessed portion (318) of the body portion (308) further comprises a plurality of protrusions (341) configured to interface with corresponding portions of the generator console (326) when the generator console is aligned with the locking members (337).

3. The generator system (400) of claim 1, further comprising a cable interface module spaced (456) apart from and communicatively coupled to the generator console, wherein the cable interface module is configured to operably couple the generator console to the neuromodulation device.

4. The generator system (300) of claim 1 wherein:
the energy delivery channel is one of a plurality of radiofrequency (RF) channels corresponding to a plurality of electrodes of the neuromodulation device; and
the display (306) is configured to numerically indicate at least one operating parameter of each RF channel in real-time during energy delivery, wherein the operating parameter is associated with a temperature measurement and/or an impedance measurement of the RF channel or wherein:
the energy delivery channel is one of a plurality of radiofrequency (RF) channels corresponding to a plurality of electrodes of the neuromodulation device; and
the display (306) is configured to provide dynamic rolling displays of at least one operating parameter of each RF channel at periodic intervals during energy delivery, wherein the operating parameter is associated with a temperature measurement and/or an impedance measurement of the RF channel.

5. The generator system (300) of claim 1 wherein the display (306) is configured to visually and/or audibly indicate when one of the operating conditions is outside of a predetermined temperature and/or impedance range, or wherein:
the energy delivery channel is one of four radiofrequency (RF) channels corresponding to four electrodes of the neuromodulation device;
the display (406) comprises a touchscreen (464), wherein the touchscreen is configured to receive input from a user to activate and/or modulate energy delivery, and wherein, during energy delivery, the display is configured to numerically and graphically indicate at least one operating parameter corresponding to each RF channel in real time;
the plurality of locking members comprise at least four latches configured to move from the unlocked arrangement to the locked arrangement within corresponding openings in the recessed portion, wherein-
the locking members are configured to automatically engage the generator console when the generator console is aligned with the locking members and received in the recessed portion, and
the locking members are configured to emit an audible sound as the locking members attach to the generator console; and
the base portion of the stand assembly includes a plurality of wheels.

6. The generator system (500) of claim 1 wherein:
the generator console is communicatively coupled to a monitor spaced apart from the generator console; and
the display (506) and the monitor (505) are configured to illustrate operating conditions of the energy delivery channel in varying levels of specificity, the display being configured to illustrate fewer details of the operating parameters than the monitor.

7. The generator system (300) of any one of claim 1, further comprising an RFID module (351) proximate to the port and configured to read an RFID tag of the neuromodulation device to identify the neuromodulation device.

## Patentansprüche

1. Generatorsystem (300, 400, 500) für eine Neuromodulationstherapie, wobei das Generatorsystem Folgendes umfasst:
eine Generatorkonsole (326), die einen Energieabgabekanal, eine Anzeige (306) und wenigstens einen Port (342) umfasst, der konfiguriert ist, um die Generatorkonsole mit einer Neuromodulationsvorrichtung (112) wirkzukoppeln, wobei die Generatorkonsole konfiguriert ist, um Energie über den Energieabgabekanal an eine Elektrode (124) der Neuromodulationsvorrichtung abzugeben, und wobei die Anzeige konfiguriert ist, um Betriebsbedingungen des Energieabgabekanals während einer Energieabgabe anzugeben; und
eine Standanordnung (304), die konfiguriert ist, um die Generatorkonsole zu tragen, **dadurch gekennzeichnet, dass** die Standanordnung Folgendes umfasst -
einen Körperabschnitt (308), der einen ausgesparten Abschnitt (318) aufweist, der konfiguriert ist, um einen Abschnitt der Generatorkonsole aufzunehmen, wobei der ausgesparte Abschnitt mehrere federbelastete Verriegelungselemente (337) beinhaltet, die von dem ausgesparten Abschnitt weg vorspringen und zwischen einer verriegelten Anordnung und einer entriegelten Anordnung bewegbar sind, und wobei die Verriegelungselemente konfiguriert sind, um an der Generatorkonsole lösbar befestigt zu werden, wenn sich die Verriegelungselemente in der verriegelten Anordnung befinden;
einen Basisabschnitt (310), der den Körperabschnitt stützt; und
einen Freigabemechanismus (335), der mit den Verriegelungselementen wirkgekoppelt ist, wobei der Freigabemechanismus konfiguriert ist, um manipuliert zu werden, um die Verriegelungselemente zwischen der verriegelten Anordnung und der entriegelten Anordnung zu bewegen, um die Generatorkonsole von der Ständeranordnung zu lösen,
wobei die Verriegelungselemente derart federbelastet sind, dass das Gewicht der Konsole auf den Verriegelungselementen die Verriegelungselemente automatisch an der Konsole einrasten kann, wenn die Konsole auf der Ständeranordnung platziert wird.

2. Generatorsystem (300) nach Anspruch 1, wobei die Verriegelungselemente (337) konfiguriert sind, um ein hörbares Geräusch zu emittieren, wenn sich die Verriegelungselemente in Verbindung mit der Generatorkonsole (326) zu der verriegelten Anordnung bewegen, oder wobei der ausgesparte Abschnitt (318) des Körperabschnitts (308) ferner mehrere Vorsprünge (341) umfasst, die konfiguriert sind, um mit entsprechenden Abschnitten der Generatorkonsole (326) eine Schnittstelle bilden, wenn die Generatorkonsole an den Verriegelungselementen (337) ausgerichtet ist.

3. Generatorsystem (400) nach Anspruch 1, das ferner ein Kabelschnittstellenmodul umfasst, das von der Generatorkonsole beabstandet (456) und kommunikativ mit dieser gekoppelt ist, wobei das Kabelschnittstellenmodul konfiguriert ist, um die Generatorkonsole mit der Neuromodulationsvorrichtung wirkzukoppeln.

4. Generatorsystem (300) nach Anspruch 1, wobei:
der Energieabgabekanal einer von mehreren Hochfrequenz(HF)-Kanälen ist, die mehreren Elektroden der Neuromodulationsvorrichtung entsprechen; und
die Anzeige (306) konfiguriert ist, um während der Energieabgabe in Echtzeit wenigstens einen Betriebsparameter jedes HF-Kanals numerisch anzugeben, wobei der Betriebsparameter einer Temperaturmessung und/oder einer Impedanzmessung des HF-Kanals zugeordnet ist oder wobei:
der Energieabgabekanal einer von mehreren Hochfrequenz(HF)-Kanälen ist, die mehreren Elektroden der Neuromodulationsvorrichtung entsprechen; und
die Anzeige (306) konfiguriert ist, um dynamische rollierende Anzeigen von wenigstens einem Betriebsparameter jedes HF-Kanals in periodischen Intervallen während der Energieabgabe bereitzustellen, wobei der Betriebsparameter einer Temperaturmessung und/oder einer Impedanzmessung des HF-Kanals zugeordnet ist.

5. Generatorsystem (300) nach Anspruch 1, wobei die Anzeige (306) konfiguriert ist, um visuell und/oder akustisch anzugeben, wenn eine der Betriebsbedingungen außerhalb eines zuvor bestimmten Temperatur- und/oder Impedanzbereichs liegt, oder wobei:
der Energieabgabekanal einer von vier Hochfrequenz(HF)-Kanälen ist, die vier Elektroden der Neuromodulationsvorrichtung entsprechen;
die Anzeige (406) einen Touchscreen (464) umfasst, wobei der Touchscreen konfiguriert ist, um eine Eingabe von einem Benutzer aufzunehmen, um die Energieabgabe zu aktivieren und/oder zu modulieren, und wobei, während der Energieabgabe, die Anzeige konfiguriert ist, um wenigstens einen Betriebsparameter numerisch und grafisch anzugeben, der jedem HF-Kanal in Echtzeit entspricht;
die mehreren Verriegelungselemente wenigstens vier Einrasteinrichtungen umfassen, die konfiguriert sind, um sich von der entriegelten Anordnung zu der verriegelten Anordnung innerhalb entsprechender Öffnungen in dem ausgesparten Abschnitt zu bewegen, wobei -
die Verriegelungselemente konfiguriert sind, um die Generatorkonsole automatisch in Eingriff zu nehmen, wenn die Generatorkonsole an den Verriegelungselementen ausgerichtet und in dem ausgesparten Abschnitt aufgenommen wird, und
die Verriegelungselemente konfiguriert sind, um einen hörbaren Ton zu emittieren, wenn sich die Verriegelungselemente an der Generatorkonsole befestigen; und
der Basisabschnitt der Ständeranordnung mehrere Räder beinhaltet.

6. Generatorsystem (500) nach Anspruch 1, wobei:
die Generatorkonsole mit einem Monitor kommunikativ gekoppelt ist, der von der Generatorkonsole beabstandet ist; und
die Anzeige (506) und der Monitor (505) konfiguriert sind, um Betriebsbedingungen des Energieabgabekanals in unterschiedlichen Spezifitätsniveaus zu veranschaulichen, wobei die Anzeige konfiguriert ist, um weniger Details der Betriebsparameter als der Monitor zu veranschaulichen.

7. Generatorsystem (300) nach Anspruch 1, das ferner ein RFID-Modul (351) nahe dem Port umfasst und konfiguriert ist, um ein RFID-Tag der Neuromodulationsvorrichtung zu lesen, um die Neuromodulationsvorrichtung zu identifizieren.

## Revendications

1. Système générateur (300, 400, 500) pour une thérapie de neuromodulation, le système générateur comprenant :
une console de générateur (326) comprenant un canal de distribution d'énergie, un dispositif d'affichage (306) et au moins un port (342) conçu pour accoupler de manière fonctionnelle la console de générateur à un dispositif de neuromodulation (112), la console de générateur étant configurée pour fournir de l'énergie par l'intermédiaire du canal de distribution d'énergie à une électrode (124) du dispositif de neuromodulation, et le dispositif d'affichage étant configuré pour indiquer les conditions de fonctionnement du canal de distribution d'énergie pendant la distribution d'énergie ; et
un ensemble support (304) conçu pour porter la console du générateur, **caractérisé en ce que** l'ensemble support comprend
une partie de corps (308) ayant une partie évidée (318) conçue pour recevoir une partie de la console du générateur, la partie évidée comportant une pluralité d'éléments de verrouillage tendus par ressort (337) faisant saillie à l'écart de la partie évidée et pouvant être déplacés entre un agencement verrouillé et un agencement déverrouillé, et les éléments de verrouillage étant conçus pour se fixer de manière amovible à la console du générateur lorsque les éléments de verrouillage sont dans l'agencement verrouillé ;
une partie de base (310) supportant la partie de corps ; et
un mécanisme de libération (335) accouplé de manière fonctionnelle aux éléments de verrouillage, le mécanisme de libération étant conçu pour être manipulé afin de déplacer les éléments de verrouillage entre l'agencement verrouillé et l'agencement déverrouillé afin de désolidariser la console de générateur de l'ensemble de support,
les éléments de verrouillage étant tendus par ressort de telle sorte que le poids de la console sur les éléments de verrouillage peut verrouiller automatiquement les éléments de verrouillage à la console lorsque la console est placée sur l'ensemble de support.

2. Système générateur (300) selon la revendication 1, dans lequel les éléments de verrouillage (337) sont conçus pour émettre un son audible lorsque les éléments de verrouillage se déplacent vers l'agencement verrouillé en relation avec la console du générateur (326), ou la partie évidée (318) de la partie de corps (308) comprenant en outre une pluralité de saillies (341) conçues pour s'interfacer avec les parties correspondantes de la console de générateur (326) lorsque la console de générateur est alignée avec les éléments de verrouillage (337).

3. Système générateur (400) selon la revendication 1, comprenant en outre un module d'interface de câble espacé (456) et couplé de manière communicative à la console de générateur, le module d'interface de câble étant conçu pour accoupler de manière fonctionnelle la console de générateur au dispositif de neuromodulation.

4. Système générateur (300) selon la revendication 1, dans lequel :
le canal de distribution d'énergie est l'un d'une pluralité de canaux radiofréquence (RF) correspondant à une pluralité d'électrodes du dispositif de neuromodulation ; et
le dispositif d'affichage (306) étant configuré pour indiquer numériquement au moins un paramètre de fonctionnement de chaque canal RF en temps réel pendant la distribution d'énergie, le paramètre de fonctionnement étant associé à une mesure de température et/ou à une mesure d'impédance du canal RF ou :
le canal de distribution d'énergie étant l'un d'une pluralité de canaux radiofréquence (RF) correspondant à une pluralité d'électrodes du dispositif de neuromodulation ; et
le dispositif d'affichage (306) étant configuré pour fournir des affichages mobiles dynamiques d'au moins un paramètre de fonctionnement de chaque canal RF à des intervalles périodiques pendant la distribution d'énergie, le paramètre de fonctionnement étant associé à une mesure de température et/ou à une mesure d'impédance du canal RF.

5. Système générateur (300) selon la revendication 1, dans lequel le dispositif d'affichage (306) est configuré pour indiquer visuellement et/ou de manière sonore lorsqu'une des conditions de fonctionnement est en dehors d'une plage de température et/ou d'impédance prédéterminée, ou :
le canal de distribution d'énergie étant l'un des quatre canaux radiofréquence (RF) correspondant à quatre électrodes du dispositif de neuromodulation ;
le dispositif d'affichage (406) comprenant un écran tactile (464), l'écran tactile étant configuré pour recevoir une entrée d'un utilisateur afin d'activer et/ou de moduler la distribution d'énergie, et, pendant la distribution d'énergie, le dispositif d'affichage étant configuré pour indiquer numériquement et graphiquement au moins un paramètre de fonctionnement correspondant à chaque canal RF en temps réel ;
la pluralité d'éléments de verrouillage comprenant au moins quatre verrous conçus pour se déplacer de l'agencement déverrouillé à l'agencement verrouillé à l'intérieur des ouvertures correspondantes dans la partie évidée,
les éléments de verrouillage étant conçus pour entrer en prise automatiquement avec la console du générateur lorsque la console du générateur est alignée avec les éléments de verrouillage et reçue dans la partie évidée, et
les éléments de verrouillage étant conçus pour émettre un son audible lorsque les éléments de verrouillage se fixent à la console du générateur ; et
la partie de base de l'ensemble support comportant une pluralité de roues.

6. Système générateur (500) selon la revendication 1, dans lequel :
la console du générateur est couplée de manière communicative à un moniteur espacé de la console du générateur ; et
le dispositif d'affichage (506) et le moniteur (505) sont configurés pour illustrer les conditions de fonctionnement du canal de distribution d'énergie avec différents niveaux de spécificité, le dispositif d'affichage étant configuré pour illustrer moins de détails des paramètres de fonctionnement que le moniteur.

7. Système générateur (300) selon l'une quelconque des revendications 1, comprenant en outre un module RFID (351) à proximité du port et configuré pour lire une étiquette RFID du dispositif de neuromodulation afin d'identifier le dispositif de neuromodulation.
